# EUROPEAN PATENT SPECIFICATION

(11) **EP 1 261 873 B1**
(45) Date of publication and mention of the grant of the patent: **17.09.2008**
(21) Application number: 01912785.1
(22) Date of filing: 16.02.2001
(51) Int. Cl.: C08J 3/075, G01N 33/543, G01N 33/566

(54) **COMPOSITIONS AND METHODS FOR SURFACE IMPRINTING**
ZUSAMMENSETZUNGEN UND VERFAHREN ZUM OBERFLÄCHENEINDRÜCKEN
COMPOSITIONS ET PROCEDES DESTINES A LA REALISATION D'EMPREINTES DE SURFACE

(30) Priority: 18.02.2000 US 507299; 18.02.2000 US 507300; 09.05.2000 US 203082 P
(43) Date of publication of application: 04.12.2002
(73) Proprietor: Aspira Biosystems, Inc., South San Francisco, CA 94080 (US)
(72) Inventor: HUANG, Chin-Shiou, San Mateo, CA 94402 (US)
(74) Representative: Brasnett, Adrian Hugh
(86) International application number: PCT/US2001/005118
(87) International publication number: WO 2001/061354

(56) References cited:
- US-A- 5 110 833
- US-A- 5 310 648
- US-A- 5 587 273
- US-A- 5 821 311
- CORMACK P A G ET AL: "Molecular imprinting: recent developments and the road ahead" REACTIVE & FUNCTIONAL POLYMERS, ELSEVIER SCIENCE PUBLISHERS BV, NL, vol. 41, no. 1-3, 15 July 1999 (1999-07-15), pages 115-124, XP004172665 ISSN: 1381-5148
- KARMALKAR ET AL.: 'Molecularly imprinted hydrogels exhibit chymotrypsin-like activity' MACROMOLECULES vol. 29, no. 4, March 1996, pages 1366 - 1368, XP002940363

## Description

### 1. FIELD OF THE INVENTION

The present invention is directed to novel molecular surface imprints. Surface imprints comprise cavities that correspond in shape to the shape of a template molecule. A substantial number of the cavities are localized at the surface of the imprint and are oriented for efficient binding. The present invention is also directed to novel methods of making surface imprints. Surface localization and/or orientation provides a greater fraction of cavities accessible for binding target molecules. Surface imprints made by this method form selective complexes with their target macromolecules. Arrays of surface imprints can be used to rapidly and inexpensively screen diverse samples.

### 2. BACKGROUND OF THE INVENTION

Conventional techniques of molecular imprinting have provided useful methods for the preparation of matrices that are capable of selectively capturing a target molecule. To prepare a molecular imprint, a matrix is formed around a template molecule. After the matrix has formed and the template molecule has been removed, the resulting molecular imprint can then be used to selectively capture the template molecule. As early as 1949, a silica gel was created that selectively bound a dye (Dickey, 1949, Proc. Natl. Acad. Sci. USA 35:227-229). Recently, an imprint prepared with phenyl-α-D-mannopyranoside was sufficiently selective to resolve a racemic mixture of the saccharide (Wulff, 1998, Chemtech 28:19-26).

Current methods form imprints of template molecules in organic polymers (Wulff, 1998, supra). To create cavities of defined shape, polymerizable molecules are bound, covalently or noncovalently, to a template molecule (Wulff, 1998, supra). The resulting complex is then copolymerized in the presence of a large amount of a cross-linking reagent (Wulff, 1998, supra). The templates are then removed, leaving cavities having defined shapes (Wulff, 1998, supra). Molecular imprints made by such a technique display selective binding for the template molecule. Molecular imprints have been used for chromatographic separation, immunoassays, chemosensors, and even catalysis (Wulff, 1998, supra).

However, failings of conventional techniques limit the broad application of molecular imprints. According to a recent review, two issues "of great importance" that limit the application of conventional molecular imprints are their limited capacity and the heterogeneity of their imprint cavities (Cormack and Mosbach, 1999, Reactive and Functional Polymers 41:115-124). When used in an assay to capture the target molecule, it is believed that the random distribution of imprint cavities throughout a conventional molecular imprint limits the access of template molecules to the imprint cavities. The majority of cavities are localized in the interior of the molecular imprint and are less accessible to the template molecule than cavities that are localized at the surface of the imprint. In particular, large molecules that cannot penetrate the matrix material of a molecular imprint can bind only at surface cavities.

The binding capacity of conventional imprints is also reduced by the random orientation of their cavities. In forming a molecular imprint by conventional techniques, the template molecules are randomly oriented within the matrix. Thus, the corresponding Molecular imprint cavities are also randomly oriented. If a particular orientation of an imprint cavity binds a target molecule more efficiently than other orientations, then only the fraction of cavities that are properly oriented will display efficient binding. The random orientation of the cavities, combined with their random distribution throughout the imprint, exacerbates the poor binding capacity of conventional molecular imprints.

Finally, conventional techniques suffer from leakage of the template molecule after formation of the imprint (Wulff, 1999, supra). When the imprint is formed, many template molecules are trapped deep within the imprint matrix. Trapped template molecules that are not removed may leak during the use of the molecular imprint. Leakage of the template molecule hinders application of conventional molecular imprints, particularly applications that involve minute amounts of a target molecule or dilute solutions. This shortcoming of conventional molecular imprints has limited their application in the pharmaceutical industry (Wulff, 1999, supra).

What is needed are novel molecular imprints that overcome the shortcomings of conventional molecular imprints. Novel molecular imprints with oriented and accessible binding cavities, and less leakage of the template molecule, will have improved capacity, specificity, and applications. Relevant disclosures of the prior art are the following.

US 5,821,311 discloses stabilisers, polymers and emulsifiers used to prepare molecular imprints. It also discloses small size molecular imprinted polymer beads.

US 5,110,833 discloses a template polymerisation technique to make synthetic enzymes and antibodies for immunological applications as alternatives to natural enzymes and antibodies. The technique involves preparation of a print molecule - monomer complex followed by polymerisation around the complex and removal of the print molecule to form a print cavity.

US 5,587,273 discloses a molecularly imprinted substrate used to detect analytes in the semiconductor field, e.g. for use as sensors. A thin film is formed on a silicon wafer by removal of a solvent from a solution of a solvent, polymer, crosslinking agent, functionalising monomer and imprinting molecule. The substrate is crosslinked and the imprinting molecule is extracted to form porous polymers with molecular imprints for analyte. The pores for analyte are embedded within the porous polymer.

The Karmalkar et al article entitled "Molecularly Imprinted Hydrogels Exhibit Chymotrypsin-Like Activity" in Macromolecules, Vol. 29, No. 4, March 1996, pages 1366-1368 discloses molecular imprinted hydrogels formed by test tube polymerisation and cut into disks on a lathe. The print molecule and cobalt were then leached out.

US 5,310,648 discloses chelating of a template molecule (e.g. a protein molecule with specially arranged amino acid residues, such as histidine amino acids which can interact with metal ions) with metal ion-containing monomers to form a complex and cross-linking of a template molecule - matrix monomer complex into a polymerised matrix. The template molecule is then extracted leaving behind an imprinted cavity within the polymerised matrix.

### 3. SUMMARY OF THE INVENTION

The shortcomings in the art are overcome by the present invention, which in one aspect provides surface imprint compositions useful for capturing, isolating, detecting, analyzing and/or quantifying molecules in a sample. Generally, the surface imprint compositions comprise a matrix material having an imprint cavity of a template molecule imprinted thereon wherein 20% or more of the imprint cavities are oriented and localized at the surface of the matrix material.

The surface imprint compositions of the invention display improved binding capacity and improved binding specificity compared to conventional molecular imprints.

The surface imprints are useful for capturing, isolating, detecting, analyzing and quantifying potentially any target molecule. Structurally, the template molecule can be identical to or similar to the target molecule. In addition, the template molecule can correspond to a portion of a larger molecule. A surface imprint of a template molecule that corresponds to a portion of a target molecule is particularly useful when the target molecule is a macromolecule. Template molecules that correspond to portions of macromolecules are described in detail in WO 01/61355 (ASPIRA BIOSYSTEMS INC).

A template molecule that corresponds to a target molecule or to a portion of a target molecule is most useful for capturing a known target molecule. However, as will be discussed more thoroughly below, an important aspect of the invention includes the ability to use the imprint compositions of the invention to isolate novel molecules from complex mixtures and/or samples. In this embodiment, a template molecule can have a structure that does not necessarily correspond to a portion of any known molecule. For instance, a template molecule could be selected from a combinatorial library. For macromolecular targets, a template molecule could have a structure that corresponds to a portion of a consensus sequence derived from a family of macromolecules. Alternatively, a template molecule might also have a random structure. A molecular imprint of a template molecule can bind a novel macromolecule if the template molecule corresponds to a portion of the novel macromolecule. An array of imprints of template molecules can be used to rapidly screen a mixture for novel macromolecules such as novel polypeptides. When the template molecules are biological polymers such as peptides, an array of imprints of the complete set of template molecules of a defined number of monomer amino acids can be used to capture most or all of the polypeptides of a mixture. Template molecules that do not necessarily correspond to a portion of any known macromolecule are described in detail in WO 01/61355, supra.

Matrix materials that can comprise the imprint compositions of the invention include substances that are capable of undergoing a physical change from a fluid state to a semi-solid or solid state. In the fluid state, matrix material molecules move easily amongst themselves, and the material retains little or no definite form. A matrix material in the fluid state can be mixed with other compounds including template molecules. In the semi-solid or solid state, the matrix material is capable of defining and retaining cavities that complement the shape of template molecules dispersed or dissolved thereon. Non-limiting examples of such matrix materials include heat sensitive hydrogels such as agarose, sol-gel materials, polymerizable monomers such as acrylamide, and mixtures of polymerizable monomers and cross-linking reagents.

The imprint compositions of the invention may take a variety of different forms. For example, they may be in the form of individual beads, disks, ellipses, or other regular or irregular shapes (collectively referred to as "beads"), or in the form of sheets. Each bead or sheet may comprise imprints of a single template molecule, or they may comprise imprints of two or more different template molecules. In one embodiment, the imprint composition comprises imprints of a plurality of different template molecules that are arranged in an array or other pattern such that their positions within the array or pattern correlate with their identities. Each position or address within the array may comprise an imprint of a single template molecule or imprints of a plurality of different template molecules, depending upon the application. Moreover, the entire array or pattern may comprise unique imprints, or may include redundancies, depending upon the application.

In another aspect, the invention provides methods of making surface imprint compositions. In one embodiment, the compositions are prepared with template molecules that are immobilized on a solid support. Preferably, the template molecules are immobilized by way of covalent attachment. While not intending to be bound by any theory of operation, template molecules that are immobilized on a solid support yield surface imprints in which a substantial number of imprint cavities are localized at or near the surface of the matrix material because the templates are not free to penetrate into the matrix material. Moreover, because the template molecules are immobilized at one point or end, the resultant imprint cavities tend to be oriented.

The solid support may have a single template molecule immobilized thereon, or a plurality of the same or different template molecules immobilized thereon. Solid substrates having a plurality of template molecules immobilized thereon in a spatially defined pattern or array are particularly convenient for preparing surface imprint arrays. The immobilized template molecule(s) may be template molecules *per se*, or may compose a larger conjugate, as will be described in more detail below.

To make a surface imprint composition according to this embodiment of the invention, a solid support having a template molecule immobilized thereon is contacted with a matrix material. As previously discussed, the matrix material comprises a compound or mixture of compounds that is capable of undergoing a change of physical state such that the resultant product is a solid or semi-solid matrix that is capable of retaining shaped cavities. The matrix material is contacted with the immobilized template molecule under conditions in which the change of physical state is effected. Changing the physical state of the compound or mixture of compounds in the presence of the template molecule results in a solid or semisolid matrix having the template molecules entrapped therein. The solid support is then removed, yielding a solid or semisolid matrix comprising cavities that correspond in shape to the template molecules. This resultant product is a surface imprint composition. If the template molecules are not removed from the matrix material with the solid support, they may be removed by washing as described in more detail below. It will be appreciated that the solid support on which the template molecule is immobilized should have dimensions that are sufficiently large such that the solid support does not become embedded within the matrix material. Preferably, the solid support will have a planar, preferably flat, surface onto which the matrix material may be poured. Non-limiting examples of such preferred solid supports include glass slides or sheets, plastic sheets, films, etc. In instances where the matrix material comprises a polymer that retains its semisolid or solid state under heat, the solid support can comprise a heat sensitive compound such as those described above. After formation of the matrix, a heat-sensitive support can be removed from the matrix by application of heat to facilitate the removal of template molecules with minimal disruption of the matrix.

According to a another embodiment, a surface imprint is prepared using a two-phase solvent system in which the template molecules are localilized at the interface of the solvents. According to this method, a conjugate molecule comprising a template moiety and a tail moiety is prepared. The template moiety constitutes the template molecule that is to be imprinted. The tail moiety constitutes a molecule that mirrors the hydrophobicity of the template molecule. For instance, if the template molecule is hydrophobic, then the tail molecule is hydrophilic. Conversely, if the template molecule is hydrophilic, then the tail molecule is hydrophobic. The template molecule and tail molecule are linked together, optionally via a linker, to form the conjugate. Due to its amphipathic nature, the conjugate molecule partitions at the interface of a two-phase system.

To make the surface imprint, the conjugate and the matrix material (in its fluid state) are mixed with a solvent system that is capable of forming two phases. When mixed or dissolved in this system, the conjugate molecule partitions at the interface of the two-phase system, with the template moiety of the conjugate residing or partitioning in one phase and the tail moiety of the conjugate residing or partitioning in the other phase. The matrix material is chosen so that it partitions into the same phase of the two-phase system as the template moiety. The mixture passively forms, or is induced to form, two phases, and conditions under which the matrix material changes from a fluid state to a semisolid or solid state are applied or effected. Changing the physical state of the matrix material in the presence of the template moiety results in a solid or semisolid matrix having the template moieties entrapped at the surface of the matrix. The tail moiety remains partitioned in the other phase of the two-phase system and does not become entrapped by the solid or semisolid matrix material. The conjugate molecules are then removed, yielding a solid or semisolid matrix comprising cavities located at or near the surface of the matrix material that correspond in shape to the template moieties. This resultant product is a surface imprint composition.

In still another aspect, the present invention provides methods of using the surface imprint compositions to capture, isolate, detect, analyze and/or quantify a molecule of interest in a sample. According to the method, a sample suspected of containing a molecule of interest is contacted with a surface imprint composition of the invention under conditions in which the molecule binds the imprint composition. The surface imprint-molecule complex may be optionally rinsed to remove unbound components of the sample. The molecule may be dissociated from the complex and isolated and/or quantified. Alternatively, the presence of the molecule may be detected, and/or its quantity determined, without dissociating it from the complex.

The methods can be used to capture molecules of known, partially known or unknown structure. In the former two embodiments, the imprint composition comprises an imprint of a template molecule that corresponds to a molecule of interest, or a portion thereof. In the latter embodiment, the imprint may comprise an imprint of a template molecule that corresponds to a conserved portion of a specific class of molecules, such as for example, a conserved portion of a receptor superfamily or family, or it may comprise an imprint of a template molecule with a novel or random structure. These latter imprint compositions can be used to capture and/or isolate novel members of known classes of molecules, or completely new types of molecules.

Molecules may be detected, captured, isolated, analyzed and/or quantified according to the methods of the invention singly, using a surface imprint composition specific for a particular molecule of interest, or alternatively, pluralities of different molecules can be captured simultaneously from a complex mixture using, for example, the array or pattern imprint compositions described herein, for subsequent detection, isolation, analysis and/or quantification.

The methods and compositions of the invention provide significant advantages over currently available molecular imprinting technologies. Unlike known imprinting techniques, the imprint cavities of the imprint compositions of the present invention are localized at the surface of the matrix material. Surface imprints are more sensitive than conventional imprints because surface imprints have a higher number of imprint cavities accessible at or near their surface for binding a target molecule. The greater density of accessible imprint cavities also reduces the amount of nonspecific binding of the imprints, further increasing the sensitivity of the surface imprints of the present invention. In particular, the surface imprints of the invention have a significantly improved capacity for binding large molecules that cannot penetrate into the matrix material.

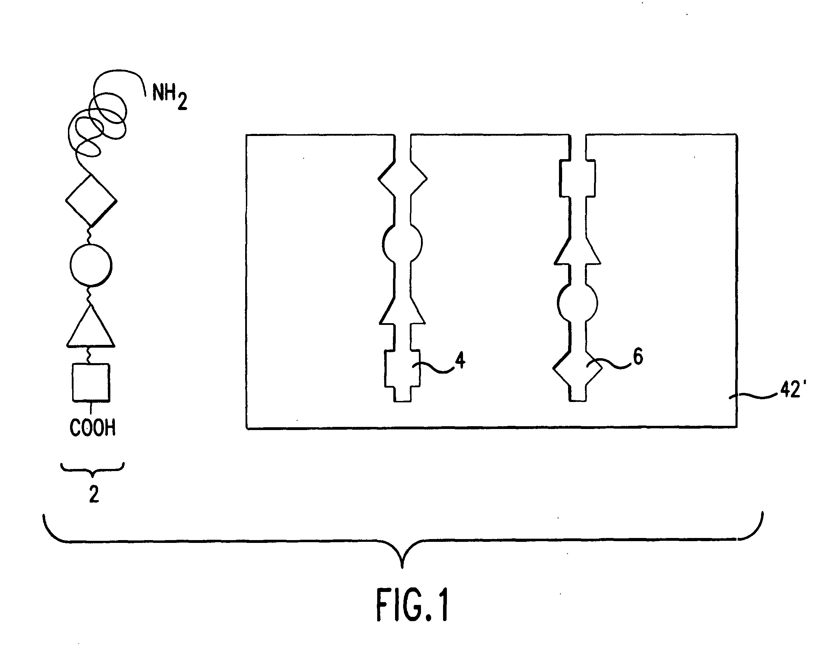

In addition, because the template molecules are oriented during the formation of the surface imprints, the surface imprints of the present invention have cavities that are oriented, as compared with random cavities obtained using conventional techniques. Proper orientation of imprint cavities can improve the binding properties of a molecular imprint. In particular, when an imprint cavity corresponds to a portion of the molecule to be captured by the imprint, then the orientation of the cavity has a significant effect on its binding efficiency. For instance, referring to FIG. 1, if imprint cavities correspond to the carboxy-terminal portion of polypeptide 2, then imprint cavity 4, which is accessible at its amino terminal end, will bind polypeptide 2 more effectively than imprint cavity 6, which is not accessible at its amino terminal end. Imprint cavity 6 might not bind macromolecule 2 at all. Surface imprints can be prepared according to the present invention so that almost every cavity is uniformly oriented to bind the target molecule.

Finally, the methods and compositions of the present application are also applicable to sensitive systems such as dilute mixtures of molecules where template leakage is a problem for conventional imprints. Compared to conventional molecular imprints that can entrap template molecules in internal cavities deep within their matrices, surface imprints retain far fewer template molecules within their solvent-accessible cavities.

The methods and surface imprint compositions of the invention have widespread applicability, ranging from the detection and/or isolation of specific molecules of interest from samples, to the capture, isolation, analysis and/or quantification of pluralities of molecules from complex mixtures for applications such as, for example, expression profiling, to the discovery of novel members of known classes of molecules and/or completely new types of molecules altogether.

### 4. BRIEF DESCRIPTION OF THE FIGURES

FIG. 1 provides an illustration comparing a properly oriented imprint cavity with an improperly oriented imprint cavity of the same template molecule;
FIG. 2 provides an illustration comparing conventional molecular imprints with the surface imprint compositions of the invention;
FIG. 3A illustrates a conjugate molecule useful for preparing a surface imprint that can capture a target molecule;
FIG. 3B illustrates a method of preparing a surface imprint by imprinting immobilized template molecules;
FIG. 3C illustrates a method of preparing a surface imprint composition of the invention utilizing a two-phase system;
FIG. 4A illustrates a one-dimensional surface imprint array;
FIG. 4B illustrates a two-dimensional array of surface imprint beads distributed on a substrate;
FIG. 4C illustrates a cross-sectional view of an embodiment of a surface imprint array;
FIG. 5 illustrates the capture of a plurality of molecules with a surface imprint array;
FIG. 6 provides an SDS-PAGE analysis of a capture and isolation experiment performed with an acrylamide surface imprint composition of the present invention;
FIG. 7 provides an SDS-PAGE analysis of two experiments capturing and isolating cytochrome c from a mixture of proteins and from a cell lysate with surface imprint compositions of the present invention;
FIG. 8 provides an SDS-PAGE analysis of an experiment capturing and isolating myoglobin with an acrylamide surface imprint composition of the present invention; and
FIG. 9 provides an SDS-PAGE analysis of an experiment capturing and isolating trypsin inhibitor with an acrylamide surface imprint composition of the present invention.

### 5. DETAILED DESCRIPTION OF THE INVENTION

Current molecular imprints lack ideal binding specificity and capacity. The specificity and capacity of current molecular imprints are impaired by the poor accessibility of many of their cavities and by the heterogeneity of those cavities. In addition, current molecular imprints suffer from constant leakage of template molecules.

The present invention provides compositions and methods that overcome these and other limitations of molecular imprinting. The invention is based, in part, on the Applicant's discovery that molecular imprints in which a substantial number or fraction of cavities are oriented and localized at the surface of the matrix material can be prepared by novel methods. The surface imprints of the present invention have cavities that are more accessible than those of prior imprints and that are properly oriented for binding a target molecule. Because the surface imprints internally trap fewer template molecules, the surface imprints compositions of the invention also exhibit less template leakage during use than conventional molecular imprints.

### 5.1 Abbreviations

As used herein, the abbreviations for the genetically encoded L-enantiomeric amino acids are conventional and are as follows:

| **Amino Acid** | **One-Letter Symbol** | **Common Abbreviation** |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

The abbreviations used for the D-enantiomers of the genetically encoded amino acids are lower-case equivalents of the one-letter symbols. For example, "R" designates L-arginine and "r" designates D-arginine. When a polypeptide sequence is represented as a series of three-letter or one-letter amino acid abbreviations, it will be understood that the left-hand direction is the amino terminal direction and the right-hand direction is the carboxy terminal direction, in accordance with standard usage and convention.

### 5.2 Surface imprints

In one aspect, the invention provides a surface imprint composition comprising a matrix material defining imprint cavities of a template molecule wherein 20% or more of the imprint cavities of the template molecule are oriented and localized at the surface of the matrix material. The cavities are oriented and localized at the surface of the matrix material. The cavities have topographies that correspond to the topography of the template molecule. The template molecule is designed or selected to generate cavities that correspond in topography to a target molecule. The surface imprint can be used to specifically capture target molecules which bind the cavities. A target molecule "binds" a cavity if it becomes entrapped or immobilized within the cavity in a specific manner such that it is specifically captured from a composition comprising it. Examples of target molecules, template molecules, and matrix materials are described in detail below.

In a surface imprint of the present invention, a substantial fraction and/or number of the imprint cavities are localized at the surface of the matrix material. By a "substantial fraction" is meant that substantially more cavities are localized at the surface of the matrix material than are located at internal regions of the matrix material. By "substantial number" is meant that substantially more cavities are localized at the surface of the matrix material than are located at the surface of imprints created by conventional means. Expressed as a fraction of the total number of cavities of the imprint the number of cavities localized at the surface of the imprint is 20% or more and can be about 20% about 25%, about 33%, about 50%, about 60%, about 70%, about 80%, about 90%, about 95%, about 99%, or about 99.9%. A surface imprint can even have substantially all, or even more, of its cavities localized at its surface, or even all of its cavities localized at its surface.

Surface imprints of the invention also have a substantially greater density of imprint cavities localized at their surfaces than conventional imprints. The methods of the present invention improve upon conventional techniques to such a degree that a far greater number of imprint cavities that are localized at the surface of the imprints and that are accessible to solvents and to target molecules. The density of the surface cavities of surface imprints can be from 10% to 100% greater than the density of cavities located at the surface of conventional imprints. Compared to conventional imprints, the density of cavities located at the surface of the surface imprints of the invention can also be from 20% to 200% greater, from 30% to 300% greater, from 40% to 400% greater, more than 100% greater, more than 200% greater, more than 300% greater, or even more than 400% greater.

The surface imprint compositions of the invention also tend to have a substantially greater number or fraction of their imprint cavities oriented with respect to the surface of the matrix material than conventional imprints. Two imprint cavities of the same or similar template molecules are "oriented" if they have a similar or identical spatial relationship to the surface of the matrix material. For example, two imprint cavities of peptides, even peptides of different primary structures, are oriented if the portions of the cavities that correspond to the amino termini of the peptides are, for instance, closer to the surface of the matrix material than the portions that correspond to the carboxy termini, or vice versa. Two imprint cavities of single-stranded polynucleotides are oriented if the portions of the cavities that correspond to the 5' ends of the polynucleotides are, for instance, closer to the surface of matrix material than the portions that correspond to the 3' ends, or vice versa. Two imprint cavities of amphipathic molecules are oriented if the portions of the cavities that correspond, for instance, to the hydrophilic portion of the molecules are closer to the surface of the matrix material than the portions that correspond to their hydrophobic portions, or vice versa. One of skill in the art will recognize that any two imprint cavities of similar template molecules can be oriented within a matrix material. Expressed as a fraction of the total number of imprint cavities, the number of oriented cavities can be about 20%, about 25%, about 33%, about 50%, about 60%, about 70%, about 80%, about 90%, about 95%, about 99%, or about 99.9%. A surface imprint can even have substantially all, or even more, of its imprinted cavities oriented with respect to the surface of the matrix material.

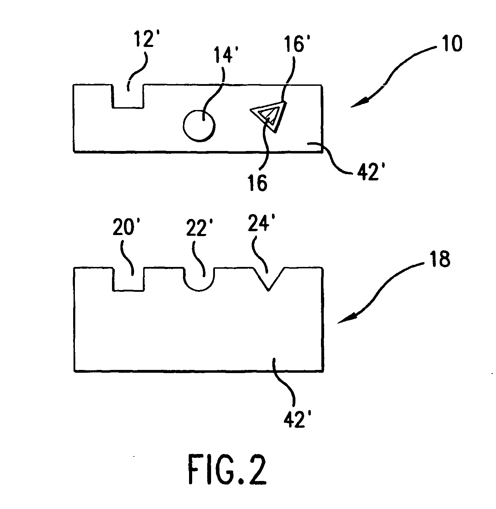

Surface imprints of the present invention are superior to conventional molecular imprints because the cavities of surface imprints are homogeneously distributed and/or oriented at the surface of the imprint. As illustrated in FIG. 2, conventional imprint **10** has cavities **12', 14'** and **16'** distributed throughout matrix material **42'.** Some of the cavities such as **12'** might be localized at the surface of matrix material **42',** but the majority of cavities such as **14'** are not surface-accessible. Some cavities such as **16'** may even contain internally trapped template molecules such as **16.** In contrast, surface imprint **18** has cavities **20', 22'** and **24'** oriented and localized at or near the surface of the matrix material. Cavities **20', 22'** and **24'** contain no trapped template molecules are accessible and oriented at the surface of the imprint.

### 5.3 Methods of making imprints

The present invention also encompasses methods of making the surface imprint compositions. Virtually any method that is capable of generating molecular imprints that have a substantial fraction or number of their imprint cavities oriented and/or localized at or near the surface of the matrix material can be used to make the surface imprint compositions of the invention. Two exemplary methods are described in detail below. Specific methods are provided in the Examples. It will be recognized by those of skill in the art that many available methods may be readily adapted according to the principles taught herein to make surface imprint compositions. In one exemplary method described below, a two phase system with conjugate molecules partitioned at its interface is used to generate surface imprints. In another exemplary method described below, a surface imprint is formed with an immobilized template molecule.

### 5.3.1 A two-phase method of preparing surface imprints

One embodiment of forming a surface imprint composition uses an amphipathic conjugate molecule in a two-phase system. The conjugate molecule partitions to the interface of the two-phase system, and a solid or semi-solid matrix is formed in one of the two phases. The resulting matrix comprises a surface imprint of the portion of conjugate molecule at the surface of the matrix defined by the interface.

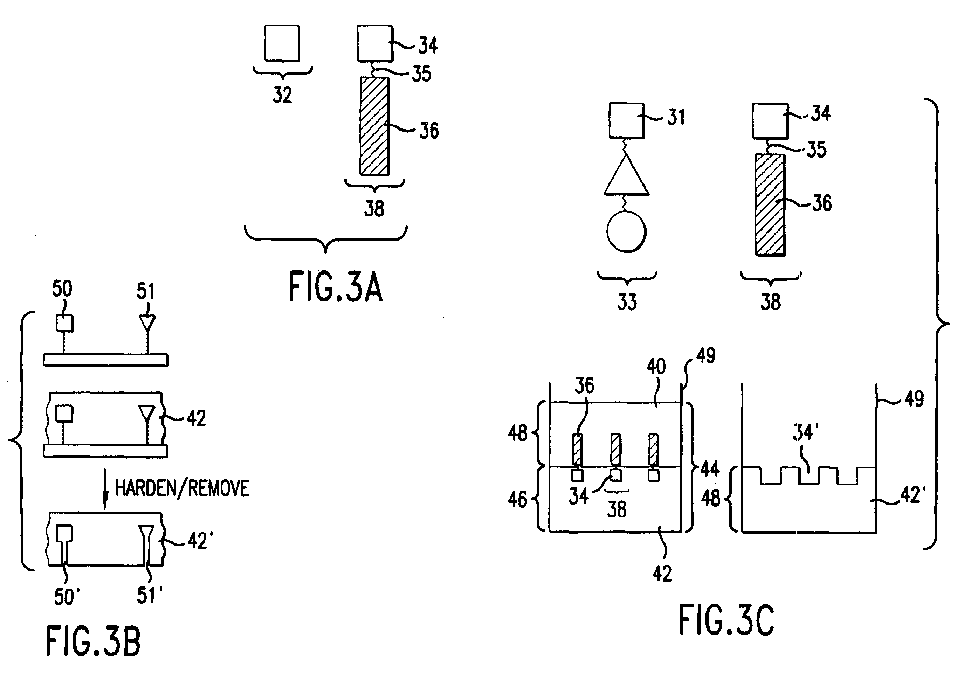

A general two-phase method for preparing a surface imprint of the present invention is illustrated in FIG. 3. Referring to FIG. 3A, to prepare a surface imprint that is useful for capturing target molecule **32,** conjugate molecule **38** is first prepared and used to form the imprint. Conjugate molecule **38** comprises a target molecule moiety **34** and a tail moiety **36.** The target molecule moiety **34** and tail moiety **36** are linked together, optionally by way of a linker **35.** As illustrated in FIG. 3A, template moiety **34** may be the target molecule to be captured **(32).** Alternatively, as illustrated in FIG. 3C, template moiety **34** may correspond to a portion **(31)** of a larger target molecule **33.** Template moieties that correspond to portions of larger target molecules, as well as methods describing how they are designed and obtained, are described in more detail, infra.

Regardless of the identity or source of template moiety **34,** tail moiety **36** has a hydrophobicity that complements, or is the opposite of, the hydrophobicity of template moiety **34.** For example, if template moiety **34** is hydrophobic, then tail moiety **36** is hydrophilic. If template moiety **34** is hydrophilic, then tail moiety **36** is hydrophobic. As a consequence, when dissolved in a two-phase solvent system in which one phase is hydrophobic and the other hydrophilic, such as an oil-and-water system, conjugate molecule **38** partitions at the interface of the two solvents. The degree of hydrophobicity or hydrophilicity of tail moiety **36** will depend on a variety of factors, including among others, the choice of solvents and the degree of hydrophobicity or hydrophilicity of template moiety **34.** Methods for determining the hydrophobicities or hydrophilicities of template moiety **34** and tail moiety **36** are described in more detail, infra. Tail moieties **36,** and optional linker moities **35** (discussed below) that yield conjugates **38** capable of partitioning at the interface of the two-phase system for particular solvents and template moieties is within the capabilities of those of skill in the art.

Template moiety **34** and tail molecule moiety **36** are linked to one another, optionally, by way of linker **35.** Specific linkers, tail moieties and template moieties are discussed in more detail, infra.

The choice of solvents used to create the two-phase system is not critical. Virtually any solvents that are immiscible with one another, that are compatible with the conjugate molecule, and that permit the conjugate molecule to partition at the interface, preferably with the template moiety residing in one phase and the tail moiety in the other, can be used.

Non-limiting examples of suitable hydrophilic (polar) solvents include water (optionally including buffering agents, salts, etc.), lower alkyl alcohols (e.g., methanol, ethanol, propanol, isopropanol, etc.), acetonitrile, dimethylsulfoxide, etc., as well as mixtures of any of these solvents.

Non-limiting examples of suitable hydrophobic (non-polar solvents) include acetone, ether, benzene, hydrocarbons such as hexane, heptane, etc., methylene chloride, carbon tetrachloride, chloroform, petroleum ether, mineral oil, phenol, etc., as well as combinations of any of the above.

The solvents may be used in a variety of different combinations to create two-phase systems. The actual choice of solvents will depend upon, among other things, the properties of the conjugate molecule. Suitable solvent systems will be apparent to those of skill in the art. Preferably, the solvents selected are non-toxic and non-teratogenic. A preferred two-phase system for most applications comprises water (or aqueous buffer) and mineral oil.

To prepare the surface imprint, as illustrated in FIG. 3B, conjugate molecule **38** is dispersed within composition **44.** Composition **44** comprises matrix material **42** and the two solvents: one hydrophobic (non-polar) and one hydrophilic (polar). As discussed above, the two solvents are immiscible such that they form a two-phase system, illustrated as first phase **46** and second phase **48.** Preferably, template moiety **34** is soluble in one of the solutions or phases (illustrated as first phase **46**) and tail moiety **36** is soluble in the other solution or phase (illustrated as second phase **48**). Matrix material **42** is also soluble in one of the two solutions or phases, typically the same solution or phase in which the template moiety **36** is soluble.

Matrix material **42** is then induced to undergo a change of physical state, to form semisolid or solid matrix **42'.** Since it is disposed throughout only one of the two phases - the phase comprising template moiety **34** - as the matrix material hardens it entraps template moiety **34.** Since the conjugate molecule **38** is localized/partitioned at the interface of the two-phase system, and the template moiety **34** is oriented in the first phase **36,** removing template moiety **34** from the "hardened" matrix material **42'** yields imprint cavities **34'** that are oriented and localized at or near the surface of hardened matrix material **42'**.

During the preparation process, composition **44** may be unagitated, thereby forming a continuous sheet of hardened matrix material **42'** defining surface imprint cavities **34'.** Alternatively, the composition may be agitated, such as by sonication or other means known to those of skill in the art. During agitation or sonication, small droplets of one phase in the other or an emulsion is obtained. Agitation or sonication thus yields an increased surface area of matrix material **42'** and a greater number of surface imprint cavities **34'** per volume of matrix material **42'.** Suitable agitation and/or sonication conditions will be apparent to one of skill in the art. For instance, sonication of an aqueous acrylamide/mineral oil mixture at 60 W for 4 min to 10 min yields a surface imprint composition having a high density of surface imprint cavities.

Matrix material **42** is a compound or mixture of compounds that is capable of undergoing a change of physical state from a fluid state to a solid or semisolid state. In the fluid state, the molecules of matrix material **42** move easily among themselves, and the material retains little or no definite form. A matrix material in the fluid state can be mixed with other compounds, including template molecules. Matrix material **42** may comprise virtually any compound or mixture of compounds that is compatible with template molecule **34** and conjugate molecule **38** and that is capable of undergoing a change of physical state to form a solid or semisolid such that the changed form is capable of retaining shaped cavities. The physical state change can be induced by virtually any means, including thermal, chemical and/or electromagnetic processes. Examples of suitable matrix materials are discussed below.

During the embedding process, matrix material **42** changes physical state, or hardens, from a fluid state to a solid or a semisolid state ("hardened") **42'** in the presence of template moiety **34.** Solid or semisolid matrix **42'** is sufficiently shape-retaining to retain imprint cavities that complement the shape of template moiety **34.** Removal of template moiety **34** from complex **46** yields surface imprint composition **48** (illustrated disposed within container **49**). In surface imprint composition **48,** solid or semisolid matrix **42'** defines cavities **34'** that complement the topography of template moiety **34.**

Although not illustrated, composition **44** can include a plurality of different conjugate molecules, each like conjugate molecule **38.** Each conjugate molecule can comprise a template moiety that corresponds to a different molecule, or a portion thereof, yielding a variation of matrix **42'** that can capture a plurality of different molecules. Alternatively, each conjugate molecule can comprise a template molecule that corresponds to a different portion of the same molecule, yielding a variation of matrix **42'** that can bind or capture the molecule at a plurality of positions.

In one two-phase embodiment, matrix material **42** is a compound or mixture of compounds that undergoes a chemical or light induced liquid-to-solid state change, such as one or more polymerizable compounds. Examples of suitable polymerizable compounds include, but are not limited to, styrene, methyl methacrylate, 2-hydroxyethyl methacrylate, 2-hydroxyethyl acrylate, methyl acrylate, acrylamide, vinyl ether, vinyl acetate, divinylbenzene, ethylene glycol dimethacrylate, ethylene glycol diacrylate, pentaerythritol dimethacrylate, pentaerythritol diacrylate, N,N'-methylenebisacrylamide, N,N'-ethylenebisacrylamide, N,N'-(1,2-dihydroxyethylene)bis-acrylamide and trimethylolpropane trimethacrylate, vinyl cyclodextrin, and polymerizable cyclodextrin. Further examples of polymerizable compounds that are useful for the preparation of surface imprints can be found in U.S. Patent No. 5,858,296. Methods for inducing polymerization of these compounds are well-known. A preferred polymerizable compound is acrylamide.

The use of such polymerizable compounds is illustrated in FIG. 3C, where the matrix material **42** is referred to in this paragraph as polymerizable compound **42.** Referring to FIG. 3C, conjugate molecule **38** and a polymerizable compound **42** are mixed in a solvent that is suitable for polymerization of polymerizable compound **42.** If necessary, an initiator for the polymerization of the polymerizable compound is included. Optionally, the conjugate molecule **38** can be covalently bound to the polymerizable compound **42,** or the two can be allowed to form non-covalent complexes. Polymerization can by started by adding an appropriate catalyst such as ultraviolet radiation. After polymerization is complete, the conjugate molecule **38** is removed by diffusion, incubation in a chaotropic reagent such as urea or guanidine, or by other techniques known to those of skill in the art.

Cross-linking reagents can be optionally used with a polymerizable compound **42** to confer rigidity to the resultant surface imprint. The present invention contemplates any ratio of polymerizable compound to cross-linking reagent that yields a surface imprint of sufficient integrity to form a cavity whose shape corresponds to the shape of the template molecule. Cross-linking reagents are known to those of skill in the art. Examples of such cross-linking reagents can be found in U.S. Patent No. 5,858,296. Preferred surface imprints are prepared with acrylamide and the cross-linking reagent ethylene glycol dimethacrylate (EGDMA).

In another embodiment, matrix material **42** is a sol-gel material. Referring to FIG. 3B, to make an imprint composition using such a sol-gel material, conjugate molecule **38** and sol-gel material **42** are mixed under conditions in which sol-gel material **42** is in a fluid or semi-fluid state. Sol-gel material **42** is allowed or induced to form a solid or a semisolid state, and the conjugate molecule **38** is removed to form hardened matrix material **42'.** In the two-phase embodiment illustrated in FIG. 3C, conjugate molecule **38** and sol-gel material **42** are mixed in a solvent that is suitable for the hardening of sol-gel material **42.** Optionally, the conjugate molecule **38** can be covalently bound to the sol-gel material **42,** or the two can be allowed to form non-covalent complexes. Sol-gel material **42** is allowed or induced to form a solid or a semisolid state **42',** and conjugate molecule **38** is removed to form imprint composition **48.** Specific conditions including solvent, temperature, catalyst, and monomer, can be selected to tailor properties of hardened matrix material **42'** or imprint composition **48** such as porosity, pore size, density, surface area and surface hydrophobicity. Sol-gel materials and detailed methods of making imprints comprising those materials can be found, for instance, in Dickey, 1949, *supra*; Dickey, 1955, J. Phys. Chem. 59: 695; Banker et al., 1982, J. Non-Crystal. Solids 48:47; Glad et al., 1985, J. Chromatography 347: 11; Wulff et al., 1986, J. Am. Chem. Soc. 108: 1089; Pinel et al., 1997, Adv. Mater 9: 582-5; U.S. Patent No. 6,057,377 and Katz & Davis, 2000, Nature 403:286-289. Preferred sol-gel materials include those that are formed from tetraethoxysilane or tetramethyloxysilane.

In general, matrix material **42** and conjugate molecule **38** can be contacted under any conditions which permit matrix material **42** to change its physical state to a solid or semisolid matrix **42'.** For instance, matrix material **42** and conjugate molecule **38** can be contacted under native conditions or under denaturing conditions. "Native conditions" and "denaturing conditions" can be defined with respect to the template moiety or with respect to the target molecule according to principles known to those of skill in the art. Preferably, the conditions under which matrix material **42** embeds template moiety **34** are similar or identical to the conditions under which molecule **32** or **33** will be captured.

The concentration of matrix material **42,** conjugate molecule **38,** and an optional cross-linking reagent can be determined according to principles known to those of skill in the art of molecular imprinting. In particular, the number of cavities **34'** in matrix **42'** can be adjusted by varying the concentration of conjugate molecule **38.** The concentration of conjugate molecule **38** can vary widely without deleteriously affecting the methods or resultant surface imprint compositions, typically from as low as 0.01 mM and as high as 1 M.

Once the matrix material **42'** is in a solid or semi-solid state, second phase **48** is removed from matrix material **42'.** Conjugate molecule **38** is removed by diffusion or by other techniques known to those of skill in the art. If conjugate molecule **38** comprises a cleavable linker **35,** then cleavable linker **35** can be cleaved to remove tail moiety **36.** Template moiety **34** can then be removed via diffusion or other techniques as previously described. Methods for removing conjugate **38** via washing are described in the Examples.

The surface imprints can take on a variety of forms. Usually, the surface imprint will initially take on the same form as the container used to create hardened matrix **42'.** However, any shape that might be useful for capturing molecules is possible. For example, the imprint compositions may be in the form of individual beads, disks, ellipses, or other regular or irregular shapes (collectively referred to as "beads"), or in the form of sheets. Beads can be formed by grinding imprint composition **48** or by suspension and dispersion techniques, as are well-known in the art. Methods of making imprinted beads are discussed in Damen et al., 1980, J. Am. Chem. Soc. 102:3265-3267; Braun et al., 1984, Chemiker-Zeitung 108:255-257; and Bystrom et al., 1993, J. Am. Chem. Soc. 115:2081-2083. Imprinted beads may also be prepared by imprinting in the pore network of preformed beaded silica as discussed in Wulff et al., 1985, Reactive Polymers 3:261-2757. Dispersion techniques are discussed in Sellergren et al., 1994, 673:133-141. The formation of beaded surface imprints by suspension polymerization is described in U.S. Patent No. 5,821,311.

### 5.3.2 Methods utilizing immobilized template molecules

In another exemplary embodiment, a surface imprint can be prepared by imprinting an immobilized template molecule or an immobilized conjugate molecule. A template molecule or conjugate molecule is first immobilized on a solid support by any technique known to those of skill in the art such as those described below. The linking group can optionally be cleavable by any means including chemicals, enzymes, or electromagnetic radiation as discussed below. The solid support can be glass, acrylic, plastic, a film, or any other substrate known to those of skill in the art.

The imprint compositions of the invention can be prepared according to any of the known techniques for making molecular imprints with one modification. Instead of creating an imprint with a free template molecule, the imprint compositions of the invention are created with an immobilized template molecule or conjugate molecule. Non-limiting examples of suitable techniques that can be used in conjunction with the invention are described, *e.g*., in U.S. Patent No. 5,858,296; U.S. Patent No. 5,786,428; U.S. Patent No. 5,587,273; U.S. Patent No. 5,821,311; U.S. Patent No. 5,814,223; and U.S. Patent No. 5,757,717; U.S. Patent No. 5,994,110; U.S. Patent No. 5,959,050; U.S. Patent No. 5,916,445; U.S. Patent No. 5,872,198; U.S. Patent No. 5,814,223; U.S. Patent No. 5,728,296; U.S. Patent No. 5,630,978; and U.S. Patent No. 5,310,648.

A general method for preparing an imprint composition of the present invention with an immobilized template molecule is illustrated in FIG. 3B. Referring to FIG. 3B, an immobilized template molecule **50** is contacted with a matrix material **42** under conditions in which template molecule **50** becomes entrapped or embedded within matrix material **42,** yielding a complex.

In this embodiment, matrix material **42** may be as described above. Additionally, matrix material **42** may be a solid or semisolid compound that liquifies upon application of heat and resolidifies when the heat is removed. Referring to FIG. 3B where matrix material **42** is referred to as "heat-sensitive compound", to make an imprint composition according to the invention using such a heat sensitive compound, conjugate molecule **38** and heat sensitive compound **42** are mixed under conditions in which heat sensitive compound **42** liquifies. The heat source is then removed and, as the liquid cools it solidifies to form complex **46.** Removal of conjugate molecules **38** *via*, for example, diffusion, yields imprint composition **48.** Of course, in order to maintain the integrity of cavities **34',** imprint composition **48** should be kept at temperatures below the liquification temperature of heat sensitive compound **42** during storage and subsequent manipulations.

Many heat-sensitive compounds that can be used to make imprint compositions according to the invention are known in the art and include, by way of example and not limitation, hydrogels such as agarose, gelatins, moldable plastics, etc. Examples of other suitable hydrogels are described in U.S. Patent No. 6,018,033; U.S. Patent No. 5,277,915, U.S. Patent No. 4,024,073, and U.S. Patent No. 4,452,892. Preferably, the temperature at which the matrix material **42** liquifies will be in a range that does not destroy or otherwise substantially degrade the conjugate molecule **38.**

During the embedding process, matrix material **42** changes physical state from a fluid state to a solid or a semisolid state **42'** in the presence of template molecule **50.** In the solid or semisolid state, matrix **42'** is sufficiently shape-retaining to retain cavities that complement the shape of template molecule **50.** Removal of the substrate and template molecule **50** yields surface imprint composition **42'** defining imprint cavity **50'.** Template molecule **50** can be removed by diffusion or by other techniques known to those of skill in the art. If template molecule **50** is immobilized by a cleavable group, template molecule **50** can preferably be cleaved to facilitate removal of the substrate and template molecule **50** from surface imprint composition **42'.**

As illustrated in FIG. 3B, a surface imprint can also be prepared by imprinting an array of template molecules. The same techniques described above are applied to an array of template molecules, such as **50** and **51,** that are immobilized on a substrate. The immobilized array of template molecules is contacted with matrix material **42** under conditions in which the array of template molecules becomes entrapped within matrix material **42.** Matrix material **42** changes physical state to a solid or semisolid matrix material **42'.** The array of template molecules is then removed yielding an array of surface imprints defining imprint cavities **50'** and **51'.** The methods of the present embodiment can be applied to any immobilized array of template molecules.

Methods for making myriad different types of immobilized template molecules are well- known. For example, methods for synthesizing peptide template molecules immobilized on synthesis substrates are described in Merrifield, 1997, Meth. Enzymol. 289:3-13; methods for synthesizing oligonucleotide template molecules immobilized on synthesis substrates are described in Southern et al., 1994, Nuc. Acids Res. 22:1368-1373. A plethora of reactions available for synthesizing a wide variety of other types of immobilized template molecules are described in Bunin, 1998, The Combinational Index, Academic Press, San Diego, CA.

The template molecule can be optionally spaced away from the solid support via a spacer molecule. The spacer molecule may be rigid, semi-rigid or flexible, hydrophilic or hydrophobic, long or short, etc. A plethora of spacers suitable for spacing molecules from solid supports are known in the art. Any of these spacers can be used to space the template molecule from the solid support. The actual choice of spacer molecule will depend upon, among other things, the nature of the template molecule, the length vs. rigidity of the spacer, etc., and will be apparent to those of skill in the art. The spacer may be selectively cleavable to aid removal of the solid support without tearing the resulting imprint composition, as will be further illustrated below.

A surface imprint composition of the present invention may also be prepared by imprinting an immobilized array of template molecules. Such arrays of immobilized template molecules can be prepared according to well-known techniques. For example, an immobilized template molecule or an immobilized array of template molecules can be prepared by spotting template molecules onto a substrate under conditions in which the template molecule is covalently or non-covalently attached to the substrate using any of the spotting devices described in U.S. Patent No. 5,601,980, U.S. Patent No. 6,001,309, U.S. Patent No. 5,785,926, and U.S. Patent No. 4,877,745. Any of these devices, or other devices useful for dispensing small aliquots of liquids into substrates, can be adapted for use to create the desired array of template molecules.

Alternatively, the array of template molecules may be prepared by synthesizing in situ each template molecule at its desired address or location within the array. Several in situ synthesis methods useful for making arrays of template molecules have been described in the art. For instance, an array of peptides immobilized on a substrate can be prepared according to, for example, U.S. Patent No. 5,958,703; U.S. Patent No. 5,919,523; U.S. Patent No. 5,847,105; or U.S. Patent No. 5,744,305. An array of oligonucleotides immobilized on a substrate can be prepared according to, for example, U.S. Patent No. 5,919,523; U.S. Patent No. 5,843,655, U.S. Patent No. 5,143,854; U.S. Patent No. 5,847,105; U.S. Patent No. 5,837,832; U.S. Patent No. 5,770,722; PCT application No. WO 92/10092; or PCT application No. WO 90/15070.

A significant advantage of preparing the arrays of the invention from template arrays is the dimensions that can be achieved. Template arrays prepared by spotting or in situ synthesis methods can readily be prepared that have synthesis spots of features on the order of 10-100 µm, permitting the synthesis of tens of thousands, or even millions, of different template molecules in a substrate area measuring about 1 cm on each edge (see, e.g., Fodor et. al., 1991, supra). Imprint arrays created with such template arrays will have similar dimension and complexities. Thus, imprint arrays capable of capturing tens of thousands, hundreds of thousands or even millions of unique macromolecules that measure only 1 cm per array axis can be readily prepared. The ability to create such miniature array imprints makes it possible, for the first time, to analyze the plethora of macromolecules present in complex samples such as cells. Due to their miniature dimensions, very little sample is required for analysis. Moreover, since template arrays at different types of template molecules can be prepared (e.g., an array comprising both peptide and oligonucleotide template molecules), different types of macromolecules can be captured and analyzed simultaneously.

In instances where the array of molecular imprints is prepared with an array of immobilized template molecules, the template molecules can be optionally spaced away from the solid support or substrate via a selectively cleavable spacer. The array of molecular imprints can then be prepared by forming imprints, according to one of the methods described above, in the presence of the array of template molecules immobilized on a support. The template molecules can then be cleaved from the support prior to removing the support from the newly formed molecular imprints. Cleavable spacers can be cleaved with chemicals, enzymes, or electromagnetic radiation. If the linkage can be cleaved with electromagnetic radiation and the transition of matrix material **42** can also be induced by electromagnetic radiation, the wavelength of the radiation that cleaves the spacer should be compatible with the wavelength of the radiation that induces the transition of the matrix material. Cleaving a labile spacer allows the template molecules to be removed from the molecular imprints, according to one of the methods described above, with minimal disruption of the integrity of the molecular imprints. The remaining portions of the template molecules can be removed by diffusion or by incubation in a chaotropic reagent such as urea or guanidine or by other techniques known to those of skill in the art for disrupting molecular complexes. Cleavable spacers suitable for attaching template molecules are known to those of skill in the art. Appropriate examples are described, for instance, in U.S. Patent No. 5,766,556; U.S. Patent No. 5,095,084; U.S. Patent No. 6,013,440; U.S. Patent No. 5,962,337; and U.S. Patent No. 5,958,703.

### 5.4 Targets

The surface imprints of the present invention can be used to detect, capture, isolate, analyze and/or quantify any target molecule. Target molecules specifically include any species that has a three-dimensional topography that is capable, at least in part, of binding cavities in a matrix material that correspond at least a portion of the three-dimensional topography of the target. Typical examples include, by way of example and not limitation, organic molecules, small molecules, therapeutic molecules, polymers, macromolecules and biological macromolecules. However, targets are not limited to molecular substances, as the surface imprints of the present invention can be used to capture substances as large as viruses and bacteria or even larger objects.

In several important embodiments, target molecules are macromolecules. Macromolecules that can be captured, isolated, detected, analyzed and/or quantified using the imprint compositions of the invention include any type of macromolecule from which a template molecule can be designed and constructed according to the principles taught herein. Virtually any type of macromolecule can be captured, isolated, detected, analyzed and/or quantified using the methods and compositions of the invention. Non-limiting examples include biological polymers such as polypeptides, polynucleotides and polysaccharides, non-biological polymers such as polyesters, polyethers, polyurethanes, block co-polymers, and other polymers known to those of skill in the art. Non-limiting examples also include biological and non-biological non-polymeric compounds such as antibiotics, steroids, natural products, dyes, etc. Thus, non-limiting examples of the myriad types of macromolecular that may be captured, isolated, detected, analyzed and/or quantified using the methods and compositions of the invention include cytokines, hormones, growth factors, enzymes, cofactors, ligands, receptors, antibodies, carbohydrates, steroids, therapeutics, antibiotics, and even larger structures such as viruses or cells, and other macromolecular targets that will be apparent to those of skill in the art.

### 5.5 Template molecules

As discussed above, the imprint compositions of the invention are prepared from a template molecule. The template molecule can be the target molecule to be captured, it can correspond to the entire structure of the target molecule, or the template molecule can correspond to a portion of the target molecule. A template molecule "corresponds" to the entire structure of the target molecule if it possesses the structural features of the target molecule as described below.

The template molecule can possess structural features of a molecule by way of structural identity with the molecule or portion. Alternatively, the template molecule can possess structural features of the molecule or portion by mimicking those structural features of the molecule. The only requirement of the template molecule is that it comprises a three-dimensional structure that is similar enough to the structure of the molecule or portion so that the molecule or portion specifically fits within a cavity formed by the template molecule.

A template molecule can correspond to a target molecule without being identical to the target molecule. Those of skill in the art will recognize that a template molecule need not have exact structural identity with the target molecule in order to "correspond" to it. Often, a template molecule may incorporate topographic substitutions. A substitution is "topographic" if the topography of the template molecule creates a cavity that binds the corresponding target molecule. Preferably, a template with a topographic substitution creates an imprint that specifically binds the corresponding target molecule. Template molecules comprising topographic substitutions, and that therefore do not correspond identically to the target molecule, are said to correspond substantially to the target molecule. Thus, unless specifically indicated otherwise, as used herein, the expression "corresponds to" is intended to encompass those situations where a template molecule corresponds identically or substantially to a molecule of interest. The correspondence between the topography of the template molecule and the topography of the target molecule should be close enough so that the target molecule fits specifically within an imprint or a cavity formed by the template molecule (see, *e*.*g*., FIG. 1).

The closeness of the correspondence between the template molecule and the molecule of interest will depend upon the desired degree of specificity between the imprint and the target molecule. Template molecules that correspond identically to the entire target molecule are expected to exhibit the highest degree of specificity for the molecule. Thus, the closeness of correspondence will depend upon the complexity of the separation, and will be apparent to those of skill in the art.

Template molecules that correspond to an entire molecule or to a portion of a molecule can be prepared according to known principles. In many instances the template molecule is simply the same molecule as the target molecule. In other instances the template molecule can be a derivative of the target molecule. For instance, the target molecule can be modified so that it can be linked to a tail molecule as described below. Alternatively, the template molecule can mimic the topography of the target molecule.

Those of skill in the art will also recognize that in many instances compounds that mimic the structures of other compounds are known. For example, peptidomimetic compounds mimic the structures of peptides. The template molecule may comprise, in whole, or in part, such mimetic structures. Mimetic compounds that can be used to create template molecules, as well as their use to create template molecules, will be apparent to those of skill in the art. All that is required is that the three dimensional surface of the mimetic template compound have a three dimensional surface with sufficient correspondence to the surface of the mimicked molecule to create a cavity that specifically fits the molecule.

If the target molecule is a macromolecule, a preferred template molecule corresponds to a portion of the macromolecule of interest. A template molecule "corresponds" to a portion of the macromolecule if it possesses the structural features of that portion of the macromolecule and substantially no other structural features of the macromolecule outside that portion. The template molecule can possess structural features of the macromolecule by way of structural identity with the portion of the macromolecule. Alternatively, the template molecule can possess structural features of the portion of the macromolecule by approximating or mimicking the structure of at least one structural moiety of the portion of the macromolecule. A detailed description of template molecules that correspond to portions of macromolecules are described in detail in copending application Serial No. 09/507,300, supra.

### 5.6 Template molecules for preparing imprints useful for capturing novel macromolecules

Template molecules that comprise the structure of a known molecule or that correspond to a portion of a known molecule are most useful for capturing known molecules. However, in another important embodiment, the present invention is also useful for capturing, isolating, detecting, analyzing, quantifying and/or identifying novel molecules. In this embodiment, the template molecule useful for preparing imprints which can capture novel molecules, even those for which no structural information is known.

In this embodiment, the template molecule can be any molecule that might be useful for capturing a novel molecule. For instance, the template molecule can be a small molecule that is useful for preparing surface imprints that can be used to capture novel molecules of similar structure. The template molecule can be selected from a combinatorial library, or any other library of molecules known to those of skill in the art. Any template molecule that can be linked to tail molecule to form a conjugate molecule, as described below, is useful for preparing surface imprints of the present invention.

In particular, template molecules of this embodiment are useful for preparing imprints that can capture a novel macromolecule. A novel macromolecule is a macromolecule for which limited or no structural or functional information is available. If any structural information is available, a molecular imprint can be prepared using a template molecule that corresponds to the portion of the available structural information as described above. The template molecule can also correspond to all of the available structural information. When no structural information is known about a macromolecule, but it is known to be functionally related to a known macromolecule, the template molecule can correspond to a portion of a macromolecule having similar function, the template molecule can correspond to a portion of a macromolecule with similar function, or the template molecule can correspond to a consensus sequence of a family of macromolecules with similar function. In addition, for any novel macromolecule, even one for which no structural or functional information is available, a molecular imprint of a template molecule with a random structure might be able to capture the novel macromolecule. For example, an as yet unidentified macromolecule can be captured, isolated, detected, analyzed and identified from a complex sample with such a molecular imprint. Template molecules appropriate for creating surface imprints that can capture novel macromolecules are described in detail in copending PCT Patent Application WO A 01/61355, supra.

### 5.7 Conjugate molecules

The conjugate molecule comprises a tail moiety and a template moiety. The conjugate molecule partitions to an interface of a multiple-phase system because of its amphipathic character. Template moieties constitute template molecules, which are described in detail above, that are to be imprinted.

Since the structure of the template moiety is determined by the structure of the target molecule, the amphipathic character of the conjugate molecule is established by careful selection of the tail moiety based on the properties of the template moiety. The tail moiety should complement the hydrophobicity of the template moiety. For example, if the template moiety is hydrophobic, then the tail moiety is hydrophilic. Alternatively, if the template moiety is hydrophilic, then the tail moiety is hydrophobic. More generally, referring to FIG. 3B, in the two-phase method, the template moiety is soluble in phase **46** which comprises matrix material **42.** The tail moiety should be chosen so that the conjugate molecule partitions to an interface of phases **46** and **48.** Preferably, the tail moiety is chosen so that it is soluble in phase **48.**

In a non-limiting example, template moiety **34** can be a peptide. The hydrophobicity of template moiety **34** can be determined according to techniques known to those of skill in the art such (Eisenberg, 1984, Ann. Rev. Biochem. 53:595-623; Eisenberg, 1984, J. Mol. Biol. 179:125-142; Eisenberg et al., 1982, Nature 299:371-374; Kyte and Doolittle, 1982, J. Mol. Biol. 157:105-32). In the instance where the template polypeptide is hydrophilic, then a hydrophobic tail moiety is chosen.

Non-limiting examples of suitable hydrophilic tail molecule moieties include molecules that bear permanent charges (*e.g*., quaternary amines and molecules comprising quaternary amines), cations derived from strong bases, anions derived from strong or organic acids (*e*.*g*., -C(O)O⁻, -P(O)₂(O⁻), -P(O)(OH)(O⁻), -O-P(O)₂(O⁻), -S(O)₂O⁻, -O-S(O)₂O⁻ and molecules comprising any of these groups or combinations thereof), polar molecules (*e*.*g*., alkyl or aryl groups substituted with one or more -OH, -SH,-NH₂, =O, -C(O)H, -C(O)OH, -C(O)O-alkyl and -C(O)NH₂ groups, polyalkylene glycols such as polyethylene glycol and polypropylene glycol, saccharides, oligosaccharides, polysaccharides, organic polymers such as polylactide and polyglycolide, organic block polymers such as PLGA, hydrophilic polypeptides such as polylysine, polynucleotides, etc.) and molecules comprising heteroatoms (*e.g*., furan, imidazole, isothiazole, isoxazole, pyrazine, etc.). The necessary degree of hydrophilicity will depend upon a variety of factors, including, among others, the solvents used to create the two-phase system and the hydrophobicity of the template molecule moiety, and will be apparent to those of skill in the art.

Non-limiting examples of suitable hydrophobic tail molecule moieties include straight-chain, branched, cyclic and polycyclic hydrocarbons, cyclic and polycyclic aryls, fatty acids such as palmitic acid, lipids, phospholipids, steroids, cholesterol and derivatives thereof, hydrophobic polypeptides, etc. The necessary degree of hydrophobicity will depend upon a variety of factors, including, among others, the solvents used to create the two-phase system and the hydrophilicity of the template molecule moiety, and will be apparent to those of skill in the art.

The template moiety is covalently linked to the tail moiety by any method known to those of skill in the art either directly or by way of an optional linker. The actual linkage will depend upon the identities of the template moiety and the tail moiety and will be apparent to those of skill in the art. For example, a hydrophilic peptide template moiety and a hydrophobic peptide tail moiety can be linked at their respective N- and C- termini to form an amide linkage. Alternatively, the template moiety and the tail moiety can be linked by a linker **35.** Linker **35** can be any molecule used by those of skill in the art to link two other molecules. Any of the spacers previously described in connection with immobilized template molecules, supra, can constitute liner **35.** Other suitable linkers **35** will be apparent to those of skill in the art. For example, bifunctional reagents are known to those of skill in the art and are commercially available. The linker molecule can form a covalent linkage between the template moiety and the tail moiety, or the linkage can be non-covalent.

The linkage between the template moiety and the tail moiety can be optionally cleavable. Cleavable linkages are known to those of skill in the art and include linkages that can be cleaved with chemicals, enzymes, or electromagnetic radiation. If the linkage can be cleaved with electromagnetic radiation and the transition of matrix material 42 can also be induced by electromagnetic radiation, the wavelength of the radiation that cleaves the linkage should be compatible with the wavelength of the radiation that induces the transition of the matrix material.

Cleavable linkages are especially useful in preparing surface imprints of an array of conjugate molecules immobilized on a solid support. Once the solid or semisolid matrix is formed, the linkage can be cleaved to facilitate removal of the solid support from the matrix with minimal disruption of the cavities embedded in the matrix. The remaining portions of the conjugate molecules can be removed by diffusion or by incubation in a chaotropic reagent such as urea or guanidine or by other techniques known to those of skill in the art for disrupting molecular complexes.

### 5.8 Arrays of surface imprints

The present invention also provides arrays of surface imprint compositions. The arrays may be comprised of a plurality of individual imprint compositions arranged in an array or pattern or may comprise a single piece or sheet of matrix having a plurality of imprint cavities imprinted thereon. In this latter embodiment, the imprint cavities are arranged in an array or pattern. The arrays may be one-dimensional, two-dimensional or three-dimensional. For instance, if the array comprises individual beads, a one-dimensional array can be prepared by introducing the beads into a capillary tube. A two-dimensional array can be prepared by distributing the beads into the wells of a microtiter plate and/or by affixing the individual beads onto a substrate.

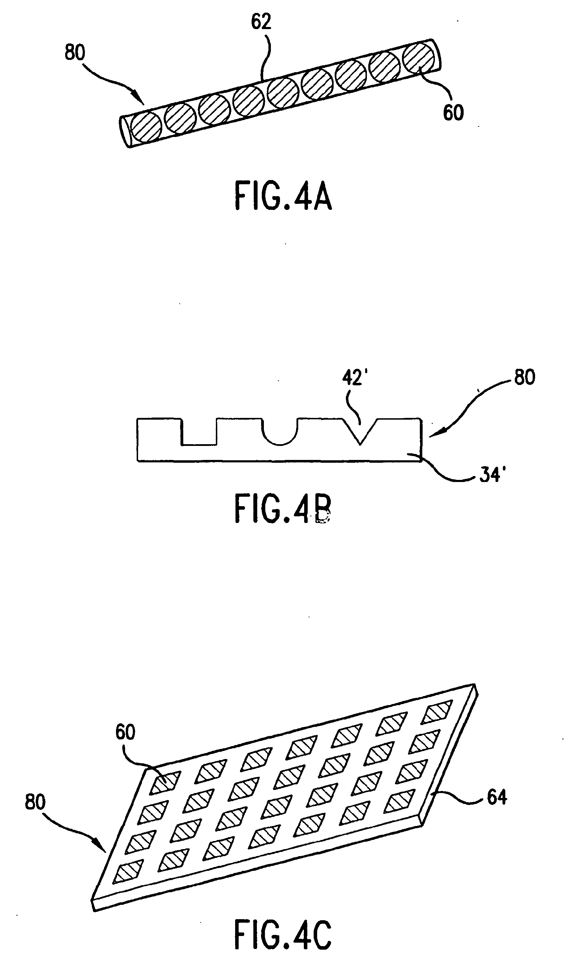

For example, referring to FIG. 4A, the array can be an ordered pattern of individual beads **60** where each bead **60** is an imprint composition of the invention. As illustrated in FIG. 4A, the individual beads **60** may be disposed within a housing **62.** Housing **62** can serve the dual purpose of retaining the ordering of individual beads **60** and providing a structure through which the sample may be flowed. The ends of the capillary tube may be optionally plugged with, for example, glass wool, a frit, or other porous materials to hold the beads in the tube during sample flow.

Alternatively, individual beads **60** could be distributed, either singly or in pluralities, amongst the wells of, for example, a microtiter plate, or affixed to the surface of a substrate, such as a glass plate, plastic sheet or film, etc. For example, referring to FIG. 4B, individual imprints **60** (in this case illustrated as square pads) can be affixed onto glass sheet **64** in an ordered two-dimensional matrix. Methods for fixing polyacrylamide pads that can be adapted to create arrays according to the invention are described in U.S. Patent No. 5,552,270. Methods of affixing other types of matrix materials onto substrates are well-known and will be apparent to those of skill in the art.

As illustrated by the above examples, a key feature of the arrays of the invention is the ability to correlate the identity of a particular imprint with its relative position within the array. Thus, in the array illustrated in FIG. 4B, the identity of a particular imprint **60** is identifiable by its xy-coordinates within the array. In the array illustrated in FIG. 4A, the identity of a particular imprint **60** is identifiable by its x-coordinate within the array. This feature is particularly useful for detecting, capturing, isolating, analyzing and/or quantifying pluralities of molecules in complex samples, as will be discussed in more detail, below.

The arrays of the invention also include matrices which have pluralities of imprint cavities disposed at defined relative positions. For example, referring to FIG. 4C, a single sheet of matrix material **42'** may comprise an ordered arrangement of imprint cavities **34'.**

In the arrays of the invention, each array element (i.e., each set of array coordinates) may be unique, i.e., each address in the array may contain an imprint of a different template molecule, or alternatively, the array may comprise redundancies. Moreover, while in many instances each array element will comprise imprint cavities of a single template molecule, one or more of the array elements may comprise imprint cavities of 2 or more different template molecules.

The number of elements comprising the array can vary over a wide range, from as few as 2 to as many as 10, 10², 10³, 10⁴, 10⁵, 10⁶ or even more, and is limited only by the ability to make an array having the desired complexity, as will be described in more detail, below.

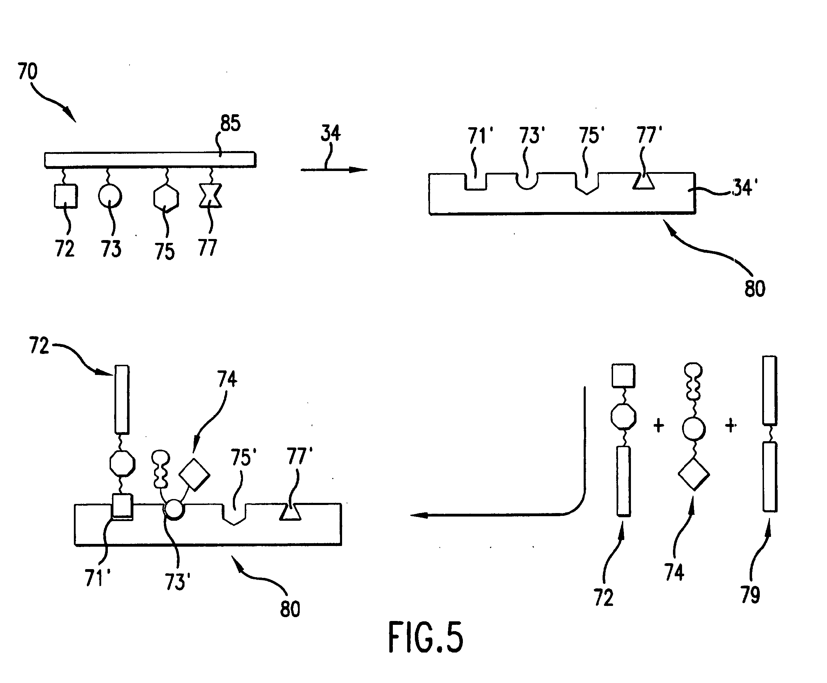

The spatially identifiable arrays of the invention provide the ability to screen and/or analyze complex samples. For example, referring to FIG. 5, imprint array **80** is constructed from template array **70.** Template array **70** comprises a plurality of template molecules **71, 73, 75** and **77** which uniquely correspond to molecules **72, 74, 76** and **78,** respectively. Imprint array **80** comprises matrix **42'** which defines cavities **71', 73', 75'** and **77'** which correspond to template molecules **71, 73, 75** and **77,** respectively Because template array **70** is spatially identifiable (i.e., the identities of the template molecules are known or identifiable by their coordinates or relative positions within the array), imprint array **80** is also spatially identifiable. Moreover, since the template molecules uniquely correspond to their respective molecules, imprint array **80** can be used to detect the presence of molecules **72, 74, 76** and **78** in a sample. For example, referring to FIG. 5, imprint array **80** is contacted with a sample comprising molecules **72, 74** and **79** under conditions in which the molecules bind their respective imprint cavities. Molecules **72** and **74** are captured at locations corresponding to cavities **71'** and **73'.** When the array is scanned for bound molecules, the presence of molecules at the addresses corresponding to templates **71** and **73** reveals that the sample contained molecules **72** and **74.** molecules **79** is not detected, as imprint array **80** does not contain an address or element capable of binding this molecule.

A spatially identifiable array of surface imprints is particularly useful when an array of surface imprints of template molecules is used to screen a complex mixture in order to isolate novel molecules. Structural information about any captured novel molecule can be deduced from the position at which it binds the array. The a portion of the captured novel molecule must have a structure that corresponds to the structure of the template molecule that was used to create the imprint at that position in the array. If the captured, novel molecule is a protein and the surface imprints is an imprint of a peptide template molecule, then a portion of the amino acid sequence of the captured protein might even be identical to the amino acid sequence of the peptide template molecule.

### 5.9 Methods of capturing target molecules

Also within the scope of the present invention are methods of using surface imprints to capture target molecules. Surface imprints useful for methods of capturing target molecules can be prepared as described above. To capture a molecule, the molecule or a mixture comprising the molecule is contacted with the molecular imprint under conditions in which the molecule binds the imprint.

Preferably, the conditions for contacting the target molecule with the imprint are similar to or identical to the conditions under which the imprint was formed. The choice of conditions depends on the target molecule and the template molecule. When the target molecule is a protein and the template molecule corresponds to sequence of amino acids of the protein, the preferred capture conditions are often denaturing. However, when a template molecule corresponds to a large fragment of a protein, such as a pepsin fragment of an immunoglobulin, then native imprinting and capture conditions will often yield superior results. When the target molecule is a double-stranded polynucleotide, the preferred capture conditions are native conditions that allow the target molecule to maintain its native structure. When the target molecule is a single-stranded polynucleotide, the capture conditions may be native or denaturing conditions. One of skill in the art will recognize whether native or denaturing conditions are appropriate. In situations where the choice of imprinting and capture conditions is not clear, the molecular imprint compositions of the present invention can be prepared so efficiently and inexpensively that a series of conditions can be assayed to determine the ideal conditions.

The exact conditions to retain a native or denatured structure are well-known and will be apparent to those of skill in the art. For instance, denaturing conditions for polypeptides can include SDS, urea, guanidine, or any other protein denaturant known to those of skill in the art. Denaturing conditions for polynucleotides can include high temperature, formamide, high ionic strength, and other conditions known to those of skill in the art.

For capture, the surface imprint compositions may be disposed within a housing to create a chromatography column, or used batch-wise. The surface imprint compositions can also be disposed in a capillary tube. Surface imprint compositions in a capillary tube can be used to capture the same target molecule. In addition, in an advantageous embodiment, surface imprint compositions in a capillary tube can be used to capture different target molecules. If the identity of each surface imprint composition in the capillary tube is known, the identity of a bound target molecule can then be determined by the position of its binding in the capillary tube.

A plurality of target molecules can be captured simultaneously by contacting the target molecules with an array of the invention. The amount of a target molecule in a sample can be quantified by capturing the target molecule with a molecular imprint and determining the amount of the target molecule captured by the imprint. Techniques for detecting a captured molecule or quantifying the amount of a captured molecule include infrared spectroscopy, UV spectroscopy, visible spectroscopy, surface acoustic wave devices, refractive index, evanescent wave sensors, bulk acoustic wave devices, capacitance measurements, radioimmunoassay measurements, radiolabeling, chemiluminescence measurements, SYPRO dyes (Steinberg et al., 1996, Anal Biochem. 239:223-37; Molecular Probes, Eugene, OR), Lamb-wave measurements, fluorescence measurements, fluorescent particles, Wilhelmy balance, chemiresistor measurements, electrochemical sensors, enzyme-linked immunosorbent assay, resistance, capacitance, acoustic wave, surface plasmon resonance, scanning tuneling microscopy, atomic force microscopy, scanning electron microscopy, rolling circle amplification, quantum dots and other techniques known to those of skill in the art for detecting or quantifying molecules such as those described in U.S. Patent No.5,306,644; U.S. Patent No. 5,313,264; U.S. Patent No. 5,955,729; and U.S. Patent No. 5,976,466.

In one representative embodiment, captured molecules can be detected or quantified by measuring the change in ultraviolet absorbence of the array of imprints before and after capture. Alternatively, the change in resistance or capacitance of the array before and after capture can be used to detect captured molecules or quantify the amount of captured molecules. In another embodiment, a plurality of macromolecules can be radioactively labeled by covalent modification with a radioactive reagent or by synthesizing the macromolecules from radioactively labeled precursors. Captured molecules can then be detected or quantified by counting radioactive emissions from the array by techniques well-known to those of skill in the art.

The relative amounts of a plurality of different target molecules can be quantified by capturing the plurality of target molecules and quantifying the amount of each target molecule of the plurality bound to the imprints. In a preferred embodiment, the identity of each imprint is determinable from its relative position within the array. An array of imprints wherein the identity of each imprint is determinable can be prepared from an array of template molecules wherein the identity of each template molecule is determinable from its relative position within the array. Methods of preparing such arrays of template molecules are known to those of skill in the art.

An array of surface imprints according to the present invention is useful for determining the relative amounts of target molecules from a complex biological source. This embodiment of the invention specifically encompasses the evaluation of an expression profile of a cell. In this embodiment, the complex mixture of target molecules comprises a plurality of polypeptides from a cell. An array of imprints is prepared using template molecules that correspond to portions of the polypeptides of the plurality. The plurality of polypeptides is contacted with the array of imprints. The absolute or relative amount of each polypeptide captured by the array of imprints is determined by a method of quantifying polypeptides known to those of skill in the art. For example, the cell that is the source of the plurality of polypeptides can be grown in the presence of radioactively labeled amino acids. The amount of each polypeptide bound by the array can then be determined by scintillation counting or by photographic exposure and densitometry. Alternatively, if antibodies are available for the polypeptides of the plurality, the amount of the polypeptides bound by the array of imprints can be determined by ELISA methods or other methods known to those of skill in the art. If each imprint of the array is on a discrete matrix, then the amount of each bound polypeptide can be determined directly by a protein assay known to those of skill in the art such as the assay described in Lowry et al., 1951, J. Biol. Chem. 193: 265-220, or the assay described in Bradford, 1976 Anal. Biochem. 72: 248-254. The expression profile of the polypeptides of the plurality can be derived from the relative amounts of each polypeptide of the plurality that is bound by the array of imprints.

A captured target molecule can be analyzed by any of the techniques discussed above for detecting or quantifying captured target molecule. For example, post-translational modification of a captured target macromolecule can be analyzed by, for instance, an ELISA method. In particular, phosphorylation of a captured target macromolecule can be analyzed with antibodies specific for phosphotyrosine or phosphoserine. Glycosylation of a captured target macromolecule can be analyzed with lectins such as concanavalin A or wheat germ agglutinin. Other features of a captured target molecule can be analyzed using any of the techniques described above or any other technique known to those of skill in the art.

A target molecule can be isolated by capturing the target molecule with a surface imprint and then recovering the target molecule from the imprint. The target molecule can be recovered from the imprint by diffusion or by incubation in urea, guanidine, SDS, or other techniques known to those of skill in the art for disrupting macromolecular complexes or for denaturing molecules.

### 5.10 Methods of screening molecules

In another aspect, the present invention is drawn to methods of screening molecules. This aspect of the invention encompasses screening both molecules of determined structure and screening of those of undetermined structure. To screen a plurality of molecules, the plurality is contacted with a plurality imprints. In one embodiment, a substrate comprises a plurality of imprints. In another embodiment, a plurality of substrates comprises a plurality of imprints.

At least one imprint of the plurality is a surface imprint of a template molecule that does not necessarily correspond to any portion of a known molecule as defined above. If the molecules to be screened are polypeptides, the template molecule should be a peptide or a polysaccharide. If the molecules to be screened are polynucleotides, the template molecule should be a polynucleotide. If the molecules to be screened are polysaccharides, the template molecule should be a polysaccharide. If the molecules to be screened are a mixture of different classes of molecules, the plurality of imprints can comprise imprints of template molecules of the corresponding classes.

A sample containing a plurality of molecules is contacted with the plurality of imprints. If any molecule of the sample contains a structure that corresponds sufficiently to the structure of the template molecule, the molecule will be captured by the plurality of imprints. Any molecules captured can be quantified or recovered from the imprint. Since template molecules can have structures that do not correspond to any portion of the structure of any known molecule, the present method of screening can be used to capture, isolate, and identify novel molecules from complex samples.

### 6. EXAMPLE 1: Preparation of a conjugate molecule comprising a template molecule corresponding to the carboxy-terminus of cytochrome c and a palmitic acid tail molecule

To create a surface imprint capable of binding the protein cytochrome c, a conjugate molecule corresponding in structure to the seven carboxy-terminal amino acids of cytochrome c was constructed. A template molecule was first designed having the amino acid sequence of the seven carboxy-terminal amino acids of the horse heart cytochrome c polypeptide, LKKATNE. A seven amino acid sequence should be sufficiently unique to provide a surface imprint with specificity for cytochrome c. A peptide with the sequence LKKATNE was synthesized by standard techniques.

A conjugate molecule was then prepared with the LKKATNE template molecule. Since LKKATNE is a hydrophilic template molecule (see Kyte & Doolittle (1982), J. Mol. Biol. 157:105-132), palmitic acid was chosen as a hydrophobic tail molecule. Palmitic acid was linked to the amino-terminus of the LKKATNE via an amide bond to form a palmitoyl-peptide conjugate molecule.

### 7. EXAMPLE 2: Preparation of an acrylamide surface imprint capable of binding cytochrome c

In this example, we demonstrate the preparation of an acrylamide surface imprint capable of binding cytochrome c. The surface imprint is prepared in a two-phase system with the conjugate molecule of Example 3 whose structure corresponds to the amino acid sequence of the carboxy-terminus of cytochrome c. The conjugate molecule, with a hydrophilic template molecule linked to a hydrophobic tail molecule, was designed to partition to the interface of the two-phase system.

Acrylamide monomer solution was prepared by dissolving 28.5 g acrylamide and 1.5 g N-N'-methylene bisacrylamide in 100 ml of 4 M urea. 2 mg of the palmitoyl-peptide conjugate molecule of Example 1 was dissolved in 1 ml of the acrylamide monomer solution. Ammonium persulfate and TEMED were added to the solution as catalysts. The final concentration of ammonium persulfate was 0.02 %, and the final concentration of TEMED was 0.1 %. 0.5 ml light mineral oil was added, and the mixture was sonicated at 60 watts for 10 min. The resulting suspension was centrifuged at 5,000xg for 10 minutes to separate phases. After polymerization at room temperature, the mineral oil phase was removed and the polymer was washed with 10 mM Tris-HCl, pH 9.0, containing 4 M urea and 10 % SDS for 24 h. The resulting matrix had the form of the interior of an Eppendorf tube.

A control polymer was prepared by the same protocol using a control conjugate molecule prepared with a control template molecule corresponding to a portion of rabbit skeletal muscle myosin heavy chain. The amino acid sequence of the control template molecule, TKVISEE, is not found in the primary amino acid sequence of horse heart cytochrome c. A palmitic acid tail molecule was linked to the amino terminus of the control template molecule via an amide bond to generate the control conjugate molecule.

### 8. EXAMPLE 3: Capture of cytochrome c with a polyacrylamide surface imprint of its C-terminal sequence

In this example we demonstrate that the acrylamide surface imprint prepared in Example 2 with a seven amino acid template molecule selectively binds the full-length cytochrome c protein.

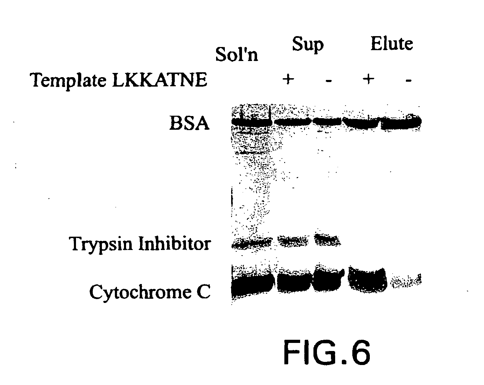

A 100 µl solution of 0.1 mg/ml bovine serum albumin, 0.1 mg/ml trypsin inhibitor, and 0.1 mg/ml cytochrome c (see FIG. 6, lane 1) in MES/urea buffer (10 mM MES, pH 5.0, and 4 M urea) was incubated with approximately 0.5 cm² surface imprint of Example 2 at room temperature for 4 h. A 100 µl sample of the same protein solution was also incubated with a control polymer prepared according to the protocol of Example 2 with the control conjugate molecule corresponding to rabbit myosin heavy chain (see FIG. 6, lanes 3 and 5). The supernatant was removed (see FIG. 6, lanes 2 and 3) and the surface imprint was washed twice with 500 ml MES/urea buffer for 15 min each. Proteins were eluted by washing overnight with 10 % SDS in MES/urea buffer (see FIG. 6, lanes 4 and 5).

The supernatant from the surface imprint incubation (see FIG. 6, lane 2) shows significantly more cytochrome c bound the surface imprint compared to the amount bound by the control polymer (see FIG. 6, lane 3). Washing with MES/urea buffer removed non-specifically bound proteins from the surface imprint and from the control polymer. Elution overnight with 10% SDS removed a fraction of the cytochrome c specifically bound to the surface imprint (see FIG. 6, lane 4) and some non-specifically bound BSA (see FIG. 6, lanes 4 and 5).

This example demonstrates that the surface imprints of the present invention can be used to specifically capture and isolate a protein from a mixture of proteins. This example also demonstrates that the capture of cytochrome c depends on the structure of the template molecule. The control polymer imprinted with a template molecule that has no correspondence to cytochrome c showed no specific binding of cytochrome c (see FIG. 6, lane 5).

### 9. EXAMPLE 4: Preparation of a second surface imprint of the C-terminal sequence of cytochrome c

In this example, we demonstrate the preparation of a second acrylamide surface imprint capable of binding cytochrome c. The surface imprint is prepared in a two-phase system with the conjugate molecule of Example I whose structure corresponds to the amino acid sequence of the carboxy-terminus of cytochrome c. The conjugate molecule, with a hydrophilic template molecule linked to a hydrophobic tail molecule, was designed to partition to the interface of the two-phase system.

Acrylamide monomer solution was prepared by dissolving 28.5 g acrylamide and 1.5 g N-N'-methylene bisacrylamide in 100 ml of 4 M urea. 2 mg of the palmitoyl-peptide conjugate molecule of Example 1 was dissolved in 1 ml of the acrylamide monomer solution. Ammonium persulfate and TEMED were added to the solution as catalysts. The final concentration of ammonium persulfate was 0.02 %, and the final concentration of TEMED was 0. %. 0.5 ml light mineral oil was added, and the mixture was sonicated at 60 watts for 4 min. The resulting suspension was centrifuged at 5,000xg for 10 minutes to separate phases. After polymerization at room temperature, the mineral oil phase was removed and the polymer was washed with 10 mM Tris-HCl, pH 9:0, containing 4 M urea and 10 % SDS for 24 h. The resulting matrix was ground into beads approximately 0.1 mm in diameter.

### 10. EXAMPLE 5: Capture of cytochrome c with a polyacrylamide surface imprint of its C-terminal sequence

In this example we demonstrate that the acrylamide surface imprint prepared in Example 4 with a seven amino acid template molecule selectively binds the full-length cytochrome c protein.

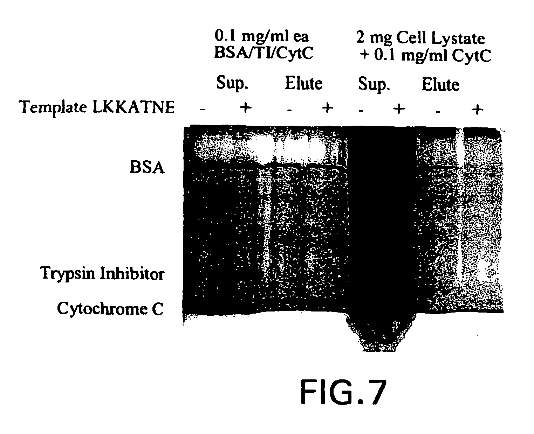

A 100 µl solution of 0.1 mg/ml bovine serum albumin, 0.1 mg/ml trypsin inhibitor, and 0.1 mg/ml cytochrome c (see FIG. 7, lane 1) in MES/urea buffer (10 mM MES, pH 5.0, and 4 M urea) was incubated with approximately 1.5 cm² surface imprint of Example 4 at room temperature for 4 h. A 100 µl sample of the same protein solution was also incubated with a control polymer prepared according to the protocol of Example 4 with no conjugate molecule (see FIG. 7, lanes 1 and 3). The supernatant was removed (see FIG. 7, lanes 1 and 2) and the surface imprint was washed twice with 500 ml MES/urea buffer for 15 min each. Proteins were eluted by washing overnight with 10 % SDS in MES/urea buffer (see FIG. 7, lanes 3 and 4).

The supernatant from the surface imprint incubation (see FIG. 7, lane 2) shows significantly more cytochrome c bound the surface imprint compared to the amount bound by the control polymer (see FIG. 7, lane 1). Washing with MES/urea buffer removed non-specifically bound proteins from the surface imprint and from the control polymer. Elution overnight with 10% SDS removed a significant fraction of the cytochrome c specifically bound to the surface imprint (see FIG. 7, lane 4) and some non-specifically bound BSA (see FIG. 7, lanes 3 and 4).

This example further demonstrates that the surface imprints of the present invention can be used to specifically capture and isolate a protein from a mixture of proteins:

### 11. EXAMPLE 6: Capture of cytochrome c from a cell lysate with a polyacrylamide surface imprint of its C-terminal sequence

In this example we demonstrate that the acrylamide surface imprint prepared in Example 4 with a seven amino acid template molecule selectively binds the full-length cytochrome c protein.

A 100 µl solution of a cell lysate (1 mg total protein from rat pheochromocytoma cells) spiked with 0.1 mg/ml cytochrome c in MES/urea buffer (10 mM MES, pH 5.0, and 4 M urea) was incubated with approximately 1.5 cm² surface imprint of Example 4 at room temperature for 4 h. A 100 µl sample of the same protein solution was also incubated with a control polymer prepared according to the protocol of Example 4 with no conjugate molecule (see FIG. 7, lanes 5 and 7). The supernatant was removed (see FIG. 7, lanes 5 and 6) and the surface imprint was washed twice with 500 ml MES/urea buffer for 15 min each. Proteins were eluted by washing overnight with 10 % SDS in MES/urea buffer (see FIG. 7, lanes 7 and 8).

The supernatants from both surface imprint compositions showed that most proteins of the cell lysate did not bind the compositions (see FIG. 7, lanes 5 and 6). Washing with MES/urea buffer removed non-specifically bound proteins from the surface imprint and from the control polymer. Elution overnight with 10% SDS removed a fraction of the cytochrome c specifically bound to the surface imprint (see FIG. 7, lane 8). The control imprint did not specifically bind cytochrome c (see FIG. 7, lane 7).

This example demonstrates the powerful specificity of the surface imprints of the present invention. The surface imprint of the carboxy-terminus selectively bound cytochrome c from a complex cell lysate. Surface imprints of the present invention can be used to capture and isolate specific molecules from the most complex mixtures of biological molecules.

### 12. EXAMPLE 7: Preparation of a conjugate molecule comprising a template molecule corresponding to the carboxy-terminus of myoglobin and a C₁₆ aliphatic chain

To create a surface imprint capable of binding myoglobin, a conjugate molecule corresponding in structure to the seven carboxy-terminal amino acids of myoglobin was constructed. A template molecule was first designed having the amino acid sequence of the seven carboxy-terminal amino acids of the horse heart myoglobin polypeptide, KELGFQG. A seven amino acid sequence should be sufficiently unique to provide a surface imprint with specificity for myoglobin. A peptide with the sequence KELGFQG was synthesized by standard techniques.

A conjugate molecule was then prepared with the KELGFQG template molecule. Since KELGFQG is a hydrophilic template molecule (see Kyte & Doolittle (1982), J. Mol. Biol. 157:105-132), palmitic acid was chosen as a hydrophobic conjugate molecule. Palmitic acid was linked to the amino-terminus of the KELGFQG via an amide bond to form a palmitoyl-peptide conjugate molecule.

### 13. EXAMPLE 8: Preparation of an acrylamide surface imprint capable of binding myoglobin

In this example, we demonstrate the preparation of an acrylamide surface imprint capable of binding myoglobin. The surface imprint is prepared in a two-phase system with the conjugate molecule of Example 7 whose structure corresponds to the amino acid sequence of the carboxy-terminus of myoglobin. The conjugate molecule, with a hydrophilic template molecule linked to a hydrophobic tail molecule, was designed to partition to the interface of the two-phase system.

Acrylamide monomer solution was prepared by dissolving 28.5 g acrylamide and 1.5 g N-N'-methylene bisacrylamide in 100 ml of 4 M urea. 2 mg of the palmitoyl-peptide conjugate molecule of Example 7 was dissolved in 1 ml of the acrylamide monomer solution. Ammonium persulfate and TEMED were added to the solution as catalysts. The final concentration of ammonium persulfate was 0.02 %, and the final concentration of TEMED was 0.1 %. 0.5 ml light mineral oil was added, and the mixture was sonicated at 60 watts for 10 min. The resulting suspension was centrifuged at 5,000xg for 10 minutes to separate phases. After polymerization at room temperature, the mineral oil phase was removed and the polymer was washed with 10 mM Tris-HCl, pH 9.0, containing 4 M urea and 10 % SDS for 24 h. The resulting matrix was ground into irregular beads.

A control polymer was prepared by imprinting a template peptide with the sequence LKKATNE as described in Example 2, supra.

### 14. EXAMPLE 9: Capture of myoglobin with a polyacrylamide surface imprint of its C-terminal sequence

In this example we demonstrate that the acrylamide surface imprint prepared in Example 8 with a seven amino acid template molecule selectively binds the full-length myoglobin protein .

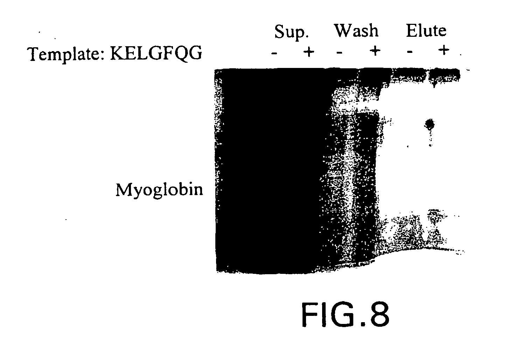

A 100 µl solution of a cell lysate (1 mg total protein from rat pheochromocytoma cells) spiked with 10 µg myoglobin in MES/urea buffer (10 mM MES, pH 5.0, and 4 M urea) was incubated with approximately 1.5 cm² surface imprint of Example 8 at room temperature for 4 h. A 100 µl sample of the same protein solution was also incubated with a control polymer prepared according to the protocol of Example 8 (see FIG. 8, lanes 2, 4 and 6). The supernatant was removed (see FIG. 8, lanes 2 and 3) and the surface imprint was washed twice with 500 ml MES/urea buffer for 15 min each (see FIG. 8, lanes 4 and 5). Proteins were eluted by washing overnight with 10 % SDS in MES/urea buffer (see FIG. 8, lanes 6 and 7).

The supernatants from both surface imprint compositions showed that most proteins of the cell lysate did not bind the compositions (see FIG. 8, lanes 2 and 3). Washing with MES/urea buffer removed non-specifically bound proteins from the surface imprint and from the control polymer (see FIG. 8, lanes 4 and 5). Elution overnight with 10% SDS removed a fraction of the myoglobin specifically bound to the surface imprint (see FIG. 8, lane 7). The control imprint did not specifically bind myoglobin (see FIG. 8, lane 6).

This example demonstrates the powerful specificity of the surface imprints of the present invention. The surface imprint of the carboxy-terminus selectively bound myoglobin from a complex cell lysate. Surface imprints of the present invention can be used to capture and isolate specific molecules from the most complex mixtures of biological molecules.

### 15. EXAMPLE 10: Preparation of a conjugate molecule comprising a template molecule corresponding to the carboxy-terminus of trypsin inhibitor and a C₁₆ aliphatic chain

To create a surface imprint capable of binding trypsin inhibitor, a conjugate molecule corresponding in structure to the seven carboxy-terminal amino acids of trypsin inhibitor was constructed. A template molecule was first designed having the amino acid sequence of the seven carboxy-terminal amino acids of the soybean trypsin inhibitor polypeptide, KLDKESL. A seven amino acid sequence should be sufficiently unique to provide a surface imprint with specificity for trypsin inhibitor. A peptide with the sequence KLDKESL was synthesized by standard techniques.

A conjugate molecule was then prepared with the KLDKESL template molecule. Since KLDKESL is a hydrophilic template molecule (see Kyte & Doolittle (1982), J. Mol. Biol. 157:105-132), palmitic acid was chosen as a hydrophobic conjugate molecule. Palmitic acid was linked to the amino-terminus of the KLDKESL via an amide bond to form a palmitoyl-peptide conjugate molecule.

### 16. EXAMPLE 11: Preparation of an acrylamide surface imprint capable of binding trypsin inhibitor

In this example, we demonstrate the preparation of an acrylamide surface imprint capable of binding trypsin inhibitor. The surface imprint is prepared in a two-phase system with the conjugate molecule of Example 10 whose structure corresponds to the amino acid sequence of the carboxy-terminus of trypsin inhibitor. The conjugate molecule, with a hydrophilic template molecule linked to a hydrophobic tail molecule, was designed to partition to the interface of the two-phase system.

Acrylamide monomer solution was prepared by dissolving 28.5 g acrylamide and 1.5 g N-N'-methylene bisacrylamide and 24 g urea in 100 ml of water. 2 mg of the palmitoyl-peptide conjugate molecule of Example 10 was dissolved in 1 ml of the acrylamide monomer solution. Ammonium persulfate and TEMED were added to the solution as catalysts. The final concentration of ammonium persulfate was 0.02 %, and the final concentration of TEMED was 0.1 %. 0.5 ml light mineral oil was added, and the mixture was sonicated at 60 watts for 10 min. The resulting suspension was centrifuged at 5,000xg for 10 minutes to separate phases. After polymerization at room temperature, the mineral oil phase was removed and the polymer was washed with 10 mM Tris-HCl, pH 9.0, containing 4 M urea and 10 % SDS for 24 h. The resulting matrix had the form of the interior of an Eppendorf tube.

A control polymer was prepared by the same protocol using a control conjugate molecule prepared with a control template molecule corresponding to a portion of rabbit muscle phosphorylase b. The amino acid sequence of the control template molecule, APDEKIP, is not found in the primary amino acid sequence of trypsin inhibitor. A palmitic acid tail molecule was linked to the amino terminus of the control template molecule via an amide bond to generate the control conjugate molecule. A second control polymer was also prepared by following the same protocol with no conjugate molecule.

### 17. EXAMPLE 12: Capture of trypsin inhibitor with a polyacrylamide surface imprint of its C-terminal sequence

In this example we demonstrate that the acrylamide surface imprint prepared in Example 11 with a seven amino acid template molecule selectively binds the full-length trypsin inhibitor protein.

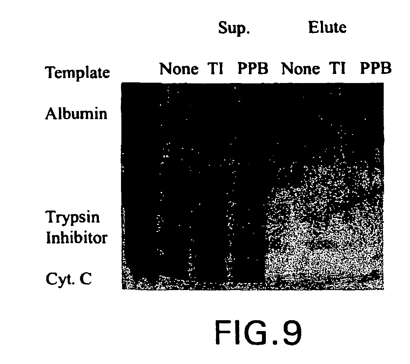

A 100 µl solution of 0.1 mg/ml bovine serum albumin, 0.1 mg/ml trypsin inhibitor, and 0.1 mg/ml cytochrome c (see FIG. 9, lane 1) in MES/urea buffer (10 mM MES, pH 5.0, and 4 M urea) was incubated with approximately 0.5 cm² surface imprint of Example 11 at room temperature for 4 h. A 100 µl sample of the same protein solution was also incubated with control polymers prepared according to the protocol of Example 11 with either no conjugate molecule (see FIG. 9, lanes 2 and 4) or the control conjugate molecule based on the carboxy terminus of phosphorylase b (see FIG. 9, lanes 4 and 7). The supernatant was removed (see FIG. 9, lanes 2, 3 and 4) and the surface imprint was washed twice with 500 ml MES/urea buffer for 15 min each. Proteins were eluted by washing overnight with 10 % SDS in MES/urea buffer (see FIG. 9, lanes 5, 6 and 7).

The supernatant from the surface imprint incubation (see FIG. 9, lane 3) shows significantly more trypsin inhibitor bound the surface imprint compared to the amount bound by the control polymers (see FIG. 9, lanes 2 and 4). Washing with MES/urea buffer removed non-specifically bound proteins from the surface imprint and from the control polymer. Elution overnight with 10% SDS removed a fraction of the trypsin inhibitor specifically bound to the surface imprint (see FIG. 9, lane 6). Trypsin inhibitor was not significantly bound by the control polymers (see FIG. 9, lanes 5 and 7).

This example demonstrates that the surface imprints of the present invention can be used to specifically capture and isolate a protein from a mixture of proteins. This example also demonstrates that the capture of trypsin inhibitor depends on the structure of the template molecule. The control polymer imprinted with a template molecule that has no correspondence to trypsin inhibitor showed no specific binding of cytochrome c (see FIG. 9, lane 7).

## Claims

1. A surface imprint composition comprising a matrix material defining imprint cavities of a template molecule wherein 20% or more of the imprint cavities of the template molecule are oriented and localized at the surface of the matrix material.

2. The surface imprint composition of claim 1 wherein 20% or 25% or 33% or 50% or 60% or 70% or 80% or 90% or 95% or 99% or 99.9% of the imprint cavities are oriented and localized at the surface of the matrix material.

3. The surface imprint composition of claim 1 or 2 in which the matrix material comprises a polymer.

4. The surface imprint composition of claim 3, wherein the polymer comprises a polymerized monomer selected from the group consisting of:
styrene,
methyl methacrylate,
2-hydroxyethyl methacrylate,
2-hydroxyethyl acrylate,
methyl acrylate,
acrylamide,
vinyl ether,
vinyl acetate,
divinylbenzene,
ethylene glycol dimethacrylate,
ethylene glycol diacrylate,
pentaerythritol dimethacrylate,
pentaerythritol diacrylate,
N,N'-methylenebisacrylamide,
N,N'-ethylenebisacrylamide,
N,N'-(1,2-dihydroxyethylene)bisacrylamide,
trimethylolpropane trimethacrylate and
vinyl cyclodextrin.

5. The surface imprint composition of claim 1, 2 or 3 in which the matrix material comprises a heat-sensitive compound.

6. The surface imprint composition of claim 5, wherein the heat-sensitive compound is selected from the group consisting of hydrogels, agarose, gelatins and moldable plastics.

7. The surface imprint composition of any one of the preceding claims, wherein the template molecule corresponds to a portion of a macromolecule of interest.

8. The surface imprint composition of any one of the preceding claims further including a macromolecule bound at an imprint cavity.

9. The surface imprint composition of claim 7 or 8, wherein the template molecule corresponds to a terminal portion of the macromolecule.

10. The surface imprint composition of any one of claims 7 to 9, wherein the macromolecule is a polynucleotide and the template molecule is an oligonucleotide.

11. The surface imprint composition of any one of claims 7 to 9, wherein the macromolecule is a polypeptide and the template molecule is an oligosaccharide.

12. The surface imprint composition of any one of claims 7 to 9, wherein the macromolecule is a polypeptide and the template molecule is a peptide.

13. The surface imprint composition of claim 12, wherein the sequence of the peptide corresponds to a contiguous sequence of the polypeptide.

14. The surface imprint composition of claim 12 or 13, wherein the peptide is between 3 and 15 amino acids in length.

15. The surface imprint composition of claim 14, wherein the peptide is between 4 and 15 amino acids in length.

16. The surface imprint composition of claim 15, wherein the peptide is between 4 and 7 amino acids in length.

17. The surface imprint composition of claim 11 or 12, wherein the portion of the polypeptide comprises the C-terminus of the polypeptide.

18. The surface imprint composition of any one of the preceding claims in which the matrix material defines imprint cavities of at least two different template molecules.

19. The surface imprint composition of claim 18 in which at least one of the template molecules corresponds to a portion of a macromolecule.

20. The surface imprint composition of claim 18 in which cavities are arranged in a spatially identifiable array.

21. A plurality of surface imprint compositions according to any one of the preceding claims.

22. The plurality of surface imprint compositions of claim 21 in which each surface imprint composition of the plurality is unique.

23. The plurality of surface imprint compositions of claim 21 in which each surface imprint composition comprises a plurality of different cavities.

24. The plurality of surface imprints of claim 21 which are arranged in a spatially identifiable array.

25. The array of claim 24 which is one-dimensional.

26. The array of claim 24 which is two-dimensional.

27. The array of claim 24 which is three-dimensional.

28. A method of preparing a surface imprint composition having imprint cavities, comprising the steps of:
(a) forming a hardened matrix in the presence of an immobilized template molecule; and
(b) removing the template molecule from the hardened matrix, yielding a surface imprint composition having 20% or more of the imprint cavities of the template molecule oriented and localized at the surface of the hardened matrix.

29. The method of claim 28 comprising forming the hardened matrix in the presence of an array of immobilized template molecules, and removing at least two of the template molecules from the hardened matrix yielding a surface imprint array capable of capturing a plurality of different molecules.

30. The method of claim 28 or 29 wherein the matrix comprises a heat sensitive compound.

31. The method of any one of claims 28 to 30 wherein the matrix comprises a polymer.

32. The method of any one of claims 28 to 31 in which the immobilization is by way of covalent attachment.

33. The method of any one of claims 28 to 32 in which the template molecule/template molecules array is immobilized via a linker molecule.

34. The method of any one of claims 28 to 33 in which the template molecule/template molecule array is immobilized on a solid support selected from the group consisting of glass, plastic and acrylic.

35. The method of any one of claims 28 to 34 in which the/each immobilized template molecule corresponds to a portion of the macromolecule of interest.

36. A method of capturing a molecule, comprising contacting the molecule with a surface imprint composition according to any one of claims 1 to 17 under conditions in which the molecule binds the surface imprint.

37. The method of claim 36 further comprising the step of recovering the molecule from the imprint to isolate the molecule.

38. The method of claim 36 further comprising the step of quantifying the amount of the molecule bound to the surface imprint in order to quantify the amount of the molecule in a sample.

39. A method of capturing a plurality of molecules, comprising contacting the plurality of molecules with a surface imprint composition according to any one of claims 18 to 20, or with a plurality or surface imprint compositions according to any one of claims 21 to 27, under conditions in which the molecules bind their corresponding surface imprint cavities.

40. The method of claim 39 further comprising the step of quantifying the amount of each molecule of the plurality bound to the plurality of surface imprints in order to quantify the relative amounts of the plurality of molecules in a sample.

41. The method of claim 38 or 40, in which the amount of the/each molecule is quantified by fluorescence, resistance, capacitance, acoustic wave, or surface plasmon resonance.

42. A method of screening for molecular structures corresponding to the structure of a template molecule from a plurality of macromolecules, comprising:
contacting the plurality of macromolecules with a surface imprint composition comprising a matrix material defining imprint cavities of the template molecule wherein 20% or more of the imprint cavities of the template molecule are oriented and localized at the surface of the matrix material and,
wherein the template molecule is selected from:
a peptide consisting of 3 to 30 amino acids,
a polynucleotide consisting of 3 to 30 nucleotides, and
an oligosaccharide consisting of 3 to 30 saccharides,
under conditions in which at least one molecule of the plurality binds the matrix.

43. The method of claim 42 comprising:
contacting the plurality of macromolecules with a plurality of surface imprint compositions, under conditions in which at least one molecule of the plurality binds one of the matrices.

## Patentansprüche

1. Oberflächenprägungszusammensetzung, umfassend ein Matrixmaterial, das Prägungshohlräume eines Matrizenmoleküls definiert, worin 20 % oder mehr der Prägungshohlräume des Matrizenmoleküls an der Oberfläche des Matrixmaterials ausgerichtet und positioniert sind.

2. Oberflächenprägungszusammensetzung nach Anspruch 1, worin 20 % oder 25 % oder 33 % oder 50 % oder 60 % oder 70 % oder 80 % oder 90 % oder 95 % oder 99 % oder 99,9 % der Prägungshohlräume an der Oberfläche des Matrixmaterials ausgerichtet und positioniert sind.

3. Oberflächenprägungszusammensetzung nach Anspruch 1 oder 2, bei der das Matrixmaterial ein Polymer umfasst.

4. Oberflächenprägungszusammensetzung nach Anspruch 3, worin das Polymer ein polymerisiertes Monomer umfasst, das aus der aus folgenden bestehenden Gruppe ausgewählt ist:
Styrol,
Methylmethacrylat,
2-Hydroxyethylmethacrylat,
2-Hydroxyethylacrylat,
Methylacrylat,
Acrylamid,
Vinylether,
Vinylacetat,
Divinylbenzol,
Ethylenglykoldimethacrylat,
Ethylenglykoldiacrylat,
Pentaerythritdimethacrylat,
Pentaerythritdiacrylat,
N,N'-Methylenbisacrylamid,
N,N'-Ethylenbisacrylamid,
N,N'-(1,2-Dihydroxyethylen-)bisacrylamid,
Trimethylolpropantrimethacrylat und
Vinylcyclodextrin.

5. Oberflächenprägungszusammensetzung nach Anspruch 1, 2 oder 3, bei der das Matrixmaterial eine wärmeempfindliche Verbindung umfasst.

6. Oberflächenprägungszusammensetzung nach Anspruch 5, worin die wärmeempfindliche Verbindung aus der aus Hydrogelen, Agarose, Gelatinen und formbaren Kunststoffen bestehenden Gruppe ausgewählt ist.

7. Oberflächenprägungszusammensetzung nach einem der vorangehenden Ansprüche, worin das Matrizenmolekül einem Abschnitt eines Makromoleküls von Interesse entspricht.

8. Oberflächenprägungszusammensetzung nach einem der vorangehenden Ansprüche, die weiters ein an einen Prägungshohlraum gebundenes Makromolekül umfasst.

9. Oberflächenprägungszusammensetzung nach Anspruch 7 oder 8, worin das Matrizenmolekül einem terminalen Abschnitt des Makromoleküls entspricht.

10. Oberflächenprägungszusammensetzung nach einem der Ansprüche 7 bis 9, worin das Makromolekül ein Polynucleotid ist und das Matrizenmolekül ein Oligonucleotid ist.

11. Oberflächenprägungszusammensetzung nach einem der Ansprüche 7 bis 9, worin das Makromolekül ein Polypeptid ist und das Matrizenmolekül ein Oligosaccharid ist.

12. Oberflächenprägungszusammensetzung nach einem der Ansprüche 7 bis 9, worin das Makromolekül ein Polypeptid ist und das Matrizenmolekül ein Peptid ist.

13. Oberflächenprägungszusammensetzung nach Anspruch 12, worin die Sequenz des Peptids einer zusammenhängenden Sequenz des Polypeptids entspricht.

14. Oberflächenprägungszusammensetzung nach Anspruch 12 oder 13, worin das Peptid eine Länge zwischen 3 und 15 Aminosäuren aufweist.

15. Oberflächenprägungszusammensetzung nach Anspruch 14, worin das Peptid eine Länge zwischen 4 und 15 Aminosäuren aufweist.

16. Oberflächenprägungszusammensetzung nach Anspruch 15, worin das Peptid eine Länge zwischen 4 und 7 Aminosäuren aufweist.

17. Oberflächenprägungszusammensetzung nach Anspruch 11 oder 12, worin der Abschnitt des Polypeptids den C-Terminus des Polypeptids umfasst.

18. Oberflächenprägungszusammensetzung nach einem der vorangehenden Ansprüche, bei der das Matrixmaterial Prägungshohlräume von zumindest zwei verschiedenen Matrizenmolekülen definiert.

19. Oberflächenprägungszusammensetzung nach Anspruch 18, bei der zumindest eines der Matrizenmoleküle einem Abschnitt eines Makromoleküls entspricht.

20. Oberflächenprägungszusammensetzung nach Anspruch 18, bei der die Hohlräume in einer räumlich identifizierbaren Anordnung angeordnet sind.

21. Vielzahl von Oberflächenprägungszusammensetzungen nach einem der vorangehenden Ansprüche.

22. Vielzahl von Oberflächenprägungszusammensetzungen nach Anspruch 21, bei denen jede Oberflächenprägungszusammensetzung der Vielzahl einzigartig ist.

23. Vielzahl von Oberflächenprägungszusammensetzungen nach Anspruch 21, bei denen jede Oberflächenprägungszusammensetzung eine Vielzahl verschiedener Hohlräume umfasst.

24. Vielzahl von Oberflächenprägungszusammensetzungen nach Anspruch 21, die in einer räumlich identifizierbaren Anordnung angeordnet sind.

25. Anordnung nach Anspruch 24, die eindimensional ist.

26. Anordnung nach Anspruch 24, die zweidimensional ist.

27. Anordnung nach Anspruch 24, die dreidimensional ist.

28. Verfahren zur Herstellung einer Oberflächenprägungszusammensetzung mit Prägungshohlräumen, umfassend folgende Schritte:
(a) das Formen einer gehärteten Matrix in Gegenwart eines immobilisierten Matrizenmoleküls; sowie
(b) das Entfernen des Matrizenmoleküls aus der gehärteten Matrix, was eine Oberflächenprägungszusammensetzung ergibt, bei der 20 % oder mehr der Prägungshohlräume des Matrizenmoleküls an der Oberfläche der gehärteten Matrix ausgerichtet und positioniert sind.

29. Verfahren nach Anspruch 28, umfassend das Formen der gehärteten Matrix in Gegenwart einer Anordnung immobilisierter Matrizenmoleküle, sowie das Entfernen von zumindest zwei der Matrizenmoleküle aus der gehärteten Matrix, was eine Oberflächenprägungsanordnung ergibt, die in der Lage ist, eine Vielzahl unterschiedlicher Moleküle einzufangen.

30. Verfahren nach Anspruch 28 oder 29, worin die Matrix eine wärmeempfindliche Verbindung umfasst.

31. Verfahren nach einem der Ansprüche 28 bis 30, worin die Matrix ein Polymer umfasst.

32. Verfahren nach einem der Ansprüche 28 bis 31, bei dem die Immobilisierung mittels kovalenten Bindens erfolgt.

33. Verfahren nach einem der Ansprüche 28 bis 32, bei dem das Matrizenmolekül / die Matrizenmolekülanordnung über ein Linkermolekül immobilisiert wird.

34. Verfahren nach einem der Ansprüche 28 bis 33, bei dem das Matrizenmolekül / die Matrizenmolekülanordnung auf einem festen Träger immobilisiert wird, der aus der aus Glas, Kunststoff und Acrylaten bestehenden Gruppe ausgewählt ist.

35. Verfahren nach einem der Ansprüche 28 bis 34, bei dem das bzw. jedes immobilisierte Matrizenmolekül einem Abschnitt des Makromoleküls von Interesse entspricht.

36. Verfahren zum Einfangen eines Moleküls, umfassend das Kontaktieren des Moleküls mit einer Oberflächenprägungszusammensetzung nach einem der Ansprüche 1 bis 17 unter Bedingungen, bei denen sich das Molekül an die Oberflächenprägung bindet.

37. Verfahren nach Anspruch 36, das weiters den Schritt des Gewinnens des Moleküls aus der Prägung umfasst, um das Molekül zu isolieren.

38. Verfahren nach Anspruch 36, das weiters den Schritt der Quantifizierung der an die Oberflächenprägung gebundenen Molekülmenge umfasst, um die Menge des Moleküls in einer Probe zu quantifizieren.

39. Verfahren zum Einfangen einer Vielzahl von Molekülen, umfassend das Kontaktieren der Vielzahl von Molekülen mit einer Oberflächenprägungszusammensetzung nach einem der Ansprüche 18 bis 20 oder mit einer Vielzahl von Oberflächenprägungszusammensetzungen nach einem der Ansprüche 21 bis 27 unter Bedingungen, bei denen sich die Moleküle an ihre entsprechenden Oberflächenprägungshohlräume binden.

40. Verfahren nach Anspruch 39, das weiters den Schritt der Quantifizierung der Menge jedes Moleküls der Vielzahl, das an die Vielzahl von Oberflächenprägungen gebunden ist, umfasst, um die relativen Mengen der Vielzahl von Molekülen in einer Probe zu quantifizieren.

41. Verfahren nach Anspruch 38 oder 40, bei dem die Menge des bzw. jedes Moleküls anhand von Fluoreszenz, elektrischem Widerstand, kapazitivem Blindwiderstand, Schallwellen oder Oberflächenplasmonresonanz quantifiziert wird.

42. Verfahren zum Screening auf der Struktur eines Matrizenmoleküls entsprechende Molekülstrukturen aus einer Vielzahl von Makromolekülen, Folgendes umfassend:
das Kontaktieren der Vielzahl von Makromolekülen mit einer Oberflächenprägungszusammensetzung, die ein Matrixmaterial umfasst, das Prägungshohlräume des Matrizenmoleküls definiert, worin 20 % oder mehr der Prägungshohlräume des Matrizenmoleküls an der Oberfläche des Matrixmaterials ausgerichtet und positioniert sind und
worin das Matrizenmolekül aus Folgendem ausgewählt ist:
einem Peptid, das aus 3 bis 30 Aminosäuren besteht,
einem Polynucleotid, das aus 3 bis 30 Nucleotiden besteht, und
einem Oligosaccharid, das aus 3 bis 30 Sacchariden besteht, unter Bedingungen, bei denen sich zumindest ein Molekül der Vielzahl an die Matrix bindet.

43. Verfahren nach Anspruch 42, umfassend:
das Kontaktieren der Vielzahl von Makromolekülen mit einer Vielzahl von Oberflächenprägungszusammensetzungen unter Bedingungen, bei denen sich zumindest ein Molekül der Vielzahl an eine der Matrizen bindet.

## Revendications

1. Composition d'empreinte de surface comprenant un matériau de matrice définissant des cavités d'empreinte d'une molécule gabarit dans laquelle 20 % ou plus des cavités d'empreinte de la molécule gabarit sont orientées et localisées au niveau de la surface du matériau de matrice.

2. Composition d'empreinte de surface selon la revendication 1, dans laquelle 20 % ou 25 % ou 33 % ou 50 % ou 60 % ou 70 % ou 80 % ou 90 % ou 95 % ou 99 % ou 99,9 % des cavités d'empreinte sont orientées et localisées au niveau de la surface du matériau de matrice.

3. Composition d'empreinte de surface selon la revendication 1 ou 2, dans laquelle le matériau de matrice comprend un polymère.

4. Composition d'empreinte de surface selon la revendication 3, dans laquelle le polymère comprend un monomère polymérisé choisi dans le groupe constitué par:
le styrène,
le méthacrylate de méthyle,
le méthacrylate de 2-hydroxyéthyle,
l'acrylate de 2-hydroxyéthyle,
l'acrylate de méthyle,
l'acrylamide,
le vinyl éther,
l'acétate de vinyle,
le divinylbenzène,
le diméthacrylate d'éthylène glycol,
le diacrylate d'éthylène glycol,
le diméthacrylate de pentaérythritol,
le diacrylate de pentaérythritol,
le N,N'-méthylènebisacrylamide,
le N,N'-éthylènebisacrylamide,
le N,N'-(1,2-dihydroxyéthylène)bisacrylamide,
le triméthacrylate de triméthylolpropane et
la vinyl cyclodextrine.

5. Composition d'empreinte de surface selon la revendication 1, 2 ou 3, dans laquelle le matériau de matrice comprend un composé thermosensible.

6. Composition d'empreinte de surface selon la revendication 5, dans laquelle le composé thermosensible est choisi dans le groupe constitué par les hydrogels, l'agarose, les gélatines et les matières plastiques moulables.

7. Composition d'empreinte de surface selon l'une quelconque des revendications précédentes, dans laquelle la molécule gabarit correspond à une partie d'une macromolécule d'intérêt.

8. Composition d'empreinte de surface selon l'une quelconque des revendications précédentes, comprenant en outre une macromolécule liée au niveau d'une cavité d'empreinte.

9. Composition d'empreinte de surface selon la revendication 7 ou 8, dans laquelle la molécule gabarit correspond à une partie terminale de la macromolécule.

10. Composition d'empreinte de surface selon l'une quelconque des revendications 7 à 9, dans laquelle la macromolécule est un polynucléotide et la molécule gabarit est un oligonucléotide.

11. Composition d'empreinte de surface selon l'une quelconque des revendications 7 à 9, dans laquelle la macromolécule est un polypeptide et la molécule gabarit est un oligosaccharide.

12. Composition d'empreinte de surface selon l'une quelconque des revendications 7 à 9, dans laquelle la macromolécule est un polypeptide et la molécule gabarit est un peptide.

13. Composition d'empreinte de surface selon la revendication 12, dans laquelle la séquence de peptide correspond à une séquence contiguë du polypeptide.

14. Composition d'empreinte de surface selon la revendication 12 ou 13, dans laquelle le peptide a une longueur comprise entre 3 et 15 acides aminés.

15. Composition d'empreinte de surface selon la revendication 14, dans laquelle le peptide a une longueur comprise entre 4 et 15 acides aminés.

16. Composition d'empreinte de surface selon la revendication 15, dans laquelle le peptide a une longueur comprise entre 4 et 7 acides aminés.

17. Composition d'empreinte de surface selon la revendication 11 ou 12, dans laquelle la partie du polypeptide comprend le terminus C du polypeptide.

18. Composition d'empreinte de surface selon l'une quelconque des revendications précédentes, dans laquelle le matériau de matrice définit des cavités d'empreinte d'au moins deux molécules gabarits différentes.

19. Composition d'empreinte de surface selon la revendication 18, dans laquelle au moins une des molécules gabarits correspond à une partie d'une macromolécule.

20. Composition d'empreinte de surface selon la revendication 18, dans laquelle les cavités sont agencées en un réseau spatialement identifiable.

21. Pluralité de compositions d'empreinte de surface selon l'une quelconque des revendications précédentes.

22. Pluralité de compositions d'empreinte de surface selon la revendication 21, dans laquelle chaque composition d'empreinte de surface de la pluralité est unique.

23. Pluralité de compositions d'empreinte de surface selon la revendication 21, dans laquelle chaque composition d'empreinte de surface comprend une pluralité de cavités différentes.

24. Pluralité d'empreintes de surface selon la revendication 21, qui sont agencées en un réseau spatialement identifiable.

25. Réseau selon la revendication 24, qui est monodimensionnel.

26. Réseau selon la revendication 24, qui est bidimensionnel.

27. Réseau selon la revendication 24, qui est tridimensionnel.

28. Procédé de préparation d'une composition d'empreinte de surface comportant des cavités d'empreinte, comprenant les étapes consistant à:
(a) former une matrice durcie en présence d'une molécule gabarit immobilisée; et
(b) retirer la molécule gabarit de la matrice durcie, donnant une composition d'empreinte de surface ayant 20 % ou plus des cavités d'empreinte de la molécule gabarit orientées et localisées au niveau de la surface de la matrice durcie.

29. Procédé selon la revendication 28, comprenant la formation de la matrice durcie en présence d'un réseau de molécules gabarits immobilisées, et le retrait d'au moins deux des molécules gabarits de la matrice durcie donnant un réseau d'empreinte de surface capable de capturer une pluralité de molécules différentes.

30. Procédé selon la revendication 28 ou 29, dans lequel la matrice comprend un composé thermosensible.

31. Procédé selon l'une quelconque des revendications 28 à 30, dans lequel la matrice comprend un polymère.

32. Procédé selon l'une quelconque des revendications 28 à 31, dans lequel l'immobilisation se fait par le biais d'une attache covalente.

33. Procédé selon l'une quelconque des revendications 28 à 32, dans lequel la molécule gabarit/le réseau de molécule gabarit est immobilisée via une molécule lieuse.

34. Procédé selon l'une quelconque des revendications 28 à 33, dans lequel la molécule gabarit/le réseau de molécule gabarit est immobilisée sur un support solide choisi dans le groupe constitué par le verre, une matière plastique et un acrylique.

35. Procédé selon l'une quelconque des revendications 28 à 34, dans lequel la/chaque molécule gabarit immobilisée correspond à une partie de la macromolécule d'intérêt.

36. Procédé de capture d'une molécule, comprenant la mise en contact de la molécule avec une composition d'empreinte de surface selon l'une quelconque des revendications 1 à 17 dans des conditions dans lesquelles la molécule se lie à l'empreinte de surface.

37. Procédé selon la revendication 36, comprenant en outre l'étape consistant à récupérer la molécule de l'empreinte pour isoler la molécule.

38. Procédé selon la revendication 36, comprenant en outre l'étape consistant à quantifier la quantité de la molécule liée à l'empreinte de surface afin de quantifier la quantité de la molécule dans un échantillon.

39. Procédé de capture d'une pluralité de molécules, comprenant la mise en contact de la pluralité de molécules avec une composition d'empreinte de surface selon l'une quelconque des revendications 18 à 20, ou avec une pluralité de compositions d'empreinte de surface selon l'une quelconque des revendications 21 à 27, dans des conditions dans lesquelles les molécules se lient à leurs cavités d'empreinte de surface correspondantes.

40. Procédé selon la revendication 39, comprenant en outre l'étape consistant à quantifier la quantité de chaque molécule de la pluralité liée à la pluralité d'empreintes de surface afin de quantifier les quantités relatives de la pluralité de molécules dans un échantillon.

41. Procédé selon la revendication 38 ou 40, dans lequel la quantité de la/chaque molécule est quantifiée par fluorescence, résistance, capacité, onde acoustique ou résonance plasmonique de surface.

42. Procédé de criblage de structures moléculaires correspondant à la structure d'une molécule gabarit provenant d'une pluralité de macromolécules, comprenant:
la mise en contact de la pluralité de macromolécules avec une composition d'empreinte de surface comprenant un matériau de matrice définissant des cavités d'empreinte de la molécule gabarit dans lequel 20 % ou plus des cavités d'empreinte de la molécule gabarit sont orientées et localisées au niveau de la surface du matériau de matrice et,
dans lequel la molécule gabarit est choisie parmi:
un peptide constitué de 3 à 30 acides aminés, un polynucléotide constitué de 3 à 30 nucléotides, et
un oligosaccharide constitué de 3 à 30 saccharides,
dans des conditions dans lesquelles au moins une molécule de la pluralité se lie à la matrice.

43. Procédé selon la revendication 42, comprenant:
la mise en contact de la pluralité de macromolécules avec une pluralité de compositions d'empreinte de surface, dans des conditions dans lesquelles au moins une molécule de la pluralité se lie à l'une des matrices.
